(19)

Europäisches
Patentamt
European
Patent Office
Office européen
des brevets

(11)     **EP 2 264 161 A1**

(12)                                **EUROPEAN PATENT APPLICATION**

(43) Date of publication:
    **22.12.2010 Bulletin 2010/51**

(51) Int Cl.:
*C12N 9/96* (2006.01)     *A61K 39/39* (2006.01)

(21) Application number: **10175575.9**

(22) Date of filing: **03.07.2006**

(84) Designated Contracting States:
    **AT BE BG CH CY CZ DE DK EE ES FI FR GB GR**
    **HU IE IS IT LI LT LU LV MC NL PL PT RO SE SI**
    **SK TR**

(30) Priority: **02.07.2005 GB 0513653**
              **22.05.2006 GB 0610140**

(62) Document number(s) of the earlier application(s) in
accordance with Art. 76 EPC:
    **06764894.9 / 1 899 462**

(71) Applicant: **Arecor Limited**
    **Cambridge CB4 OFE (GB)**

(72) Inventor: **Jezek, Jan**
    **Cambridge, Cambridgeshire CB4 0FE (GB)**

(74) Representative: **Teuten, Andrew John**
    **Sagittarius IP**
    **Taylor House**
    **39 High Street**
    **Marlow, Bucks SL7 2AF (GB)**

Remarks:
    This application was filed on 07-09-2010 as a
    divisional application to the application mentioned
    under INID code 62.

(54)     **Stable aqueous systems comprising proteins**

(57)     An aqueous system comprising a protein and two or more stabilising agents, **characterised in that** (i) the two or more stabilising agents have ionisable groups capable of exchanging protons with the protein and with the ionised products of water dissociation; (ii) wherein a first stabilising agent has first ionisable groups that are positively charged when protonated and uncharged when deprotonated, and a second stabilising agent has second ionisable groups that are uncharged when protonated and negatively charged when deprotonated, wherein the first and second ionisable groups have pKa values respectively 1 to 5 units higher and 1 to 5 units lower than the pH of the composition, and wherein the second stabilising agent is a carboxylic acid other than an amino acid; and (iii) the pH of the composition is within a range of protein stability that is at least 50% of the maximum stability of the protein with respect to pH.

**Fig. 1.**

**Description**

<u>Field of the Invention</u>

This invention relates to stable aqueous systems comprising proteins.

<u>Background of the Invention</u>

The loss of a protein's native tertiary structure is generally associated with the loss of its biological activity. It is therefore crucial to ensure that an active protein (e.g. vaccine, therapeutic protein, diagnostic protein etc.) is stored under conditions where the native tertiary structure is maintained.

Storage of proteins for any length of time poses stability problems. The fluctuations of the tertiary structure are proportional to the temperature. Proteins are therefore generally more stable at lower temperatures. Typically, proteins have to be stored freeze-dried (lyophilised) or frozen (around -20°C) to preserve their biological activity. If stored freeze-dried or frozen, the protein has to be reconstituted before its use. For short-term storage of proteins, refrigeration at 4°C may be sufficient.

Proteins are macromolecules consisting of sequences of 20 different naturally occurring amino acids. Seven of these amino acids (aspartic acid, glutamic acid, histidine, cysteine, tyrosine, lysine and arginine) contain a side-chain capable of engaging in acid-base equilibria. This means that they can either accept or donate a proton depending on pH and other species present in the solution. Serine and threonine are also capable of exchanging protons with the surrounding molecules. However, the $pK_a$ values of these amino acids are extremely high (>13.9), so their side-chains are practically always in almost totally protonated form.

EP0513914 discloses the stabilisation of uricase with aspartic acid at a concentration of 750 mM. US3051627 discloses chymotrypsin in water with an amino acid, at pH 4.

<u>Summary of the Invention</u>

According to a first aspect of the present invention, an aqueous system comprises a protein and one or more stabilising agents, **characterised in that**

   (i) the one or more stabilising agents have ionisable groups capable of exchanging protons with the protein and with the ionised products of water dissociation;
   (ii) the ionisable groups include first groups that are positively charged when protonated and uncharged when deprotonated, and second groups that are uncharged when protonated and negatively charged when deprotonated; and
   (iii) the pH of the composition is within a range of protein stability that is at least 50% of the maximum stability of the protein with respect to pH.

According to a second aspect of the invention, an aqueous system comprises, a protein and one or more stabilising agents, **characterised in that**

   (i) the one or more stabilising agents have ionisable groups capable of exchanging protons with the protein and with the ionised products of water dissociation;
   (ii) the ionisable groups include first groups that are positively charged when protonated and uncharged when deprotonated, and second groups that are uncharged when protonated and negatively charged when deprotonated; and
   (iii) the pH of the composition is within a range of $\pm 0.5$ pH units of the pH at which the composition has maximum stability with respect to pH.

According to a third aspect of the invention, a composition comprises a protein and one or more stabilising agents as defined above, adsorbed on a solid.

The present invention also provides a method of identifying an appropriate pH to optimise the storage stability of a protein in aqueous environment, comprising determining a pH with a value in the range 4 to 9 at or near a value at which the protein is in a proton-exchange equilibrium state with respect to its surroundings that is optimised for storage stability, i.e. in a condition of optimum proton-exchange equilibrium.

The optimum proton-exchange equilibrium state exists at a pH at which the following two conditions are met:

   a) the probability of protonation (P) of each individual amino acid in the protein sequence (as defined by the $pK_a$ of

each individual amino acid) is as far away as possible from 50% (i.e. 50% in protonated form and 50% in deprotonated form); and

b) the overall protonation of all amino acids in the protein sequence (as defined by $pK_a$ values of all amino acids in the sequence) is as high as possible.

The optimum proton-exchange equilibrium state, i.e. the state at which storage stability is optimised, is that in which conditions a) and b) are both met as far as possible, with a compromise being reached between the two conditions for overall optimisation.

The values of the criteria in conditions a) and b) are both dependent on pH and can be calculated for any protein at any pH. Calculation of condition a) requires a knowledge of the amino acid sequence of the protein (this may be based on all amino acids, but is preferably based on only those amino acids accessible at the surface of the protein in folded, active condition) and the $pK_a$ values of the amino acid side-chains of the protein. Only the 7 protonatable amino acids are of relevance. Calculation of condition b) also requires a knowledge of the isoelectric point of the protein. In this way, the pH at which conditions a) and b) are both met as far as possible can be determined.

In particular, the probability of protonation (P), which is also referred to herein as the proton exchange frequency, at any pH can be calculated as described above and plotted against pH to enable determination of the pH (in the interval 4 to 9) at which P is at a minimum ($P_{minimum}$).

The probability of protonation (P) can be calculated by the following mathematical function:

$$ P = \frac{100000}{M} \times \sum_{sidechains}^{All} (N \times [HA] \times [A]) $$

where:

$$ [HA] = \frac{1}{1 + \frac{10^{-pKa}}{10^{-pH}}} \qquad\qquad [A] = \frac{10^{-pKa}}{10^{-pKa} + 10^{-pH}} $$

M is the relative molecular weight of the protein unit;

N is the number of the given amino acid side-chains in the protein unit; and

$pK_a$ is the negative of the logarithm of the acid dissociation constant $K_a$ of the given amino acid side-chain.

Adjustment to take account of condition b) can then be made by calculating an optimised value of P ($P_{optimised}$) by the following equation:

$$ P_{optimised} = P_{minimum} + (A \times pI) + B $$

where:

pI is the isoelectric point of the protein; and

A and B are constants with the values A = -1.192 and B = 10.587 if the total amino acid composition is used, and A = -0.931 and B = 8.430 if only the amino acids accessible at the protein surface are used.

The pH value corresponding to $P_{optimised}$ that is lower than the pH value corresponding to that of $P_{minimum}$ can then be determined, giving the calculated optimum pH for the protein.

Use of this algorithm enables reasonably accurate prediction of an optimum pH for storage stability benefits for a particular protein. The exact optimum pH can then be determined experimentally for any given system, possibly including one or more additives as discussed above.

References to pH being "at or near" a value usually mean within $\pm 0.5$ pH units, preferably $\pm 0.4$ pH units, more preferably $\pm 0.2$ pH units.

Data for isoelectric point values and amino acid sequences, including accessibility at the surface of amino acids in the protein in folded, active condition, for many proteins is readily available from various sources, with one convenient source for sequence data being the Protein Data Bank information databank which is available on-line at www.rcsb.org/pdb. Other, paper-based sources of information are also available.

The invention also provides a method of treating a protein in aqueous environment in initial conditions of non-optimum storage stability to make the storage stability closer to optimum, comprising adjusting the pH to a value in the range 4 to 9 at or near a value at which the protein is in a proton-exchange equilibrium state with respect to its surroundings that is optimised for storage stability.

The invention additionally provides a method of providing a protein in aqueous environment with optimised conditions for storage stability, comprising providing a pH in the range 4 to 9 at or near a value at which the protein is in a proton-exchange equilibrium state with respect to it surroundings that is optimised for storage stability.

The invention also includes within its scope a protein in aqueous environment at a pH in the range 4 to 9 at or near a value at which the protein is in a proton-exchange equilibrium state with respect to its surroundings that is optimised for storage stability.

The stabilised protein may be in a microbiologically sterile form, and is conveniently contained or stored in a sealed, sterile container such as a vial, syringe or capsule.

The invention also provides a method of storage of a protein in aqueous environment, including at ambient temperature and above, comprising providing the environment with a pH in the range 4 to 9 at or near a value at which the protein is in a proton-exchange equilibrium state with respect to its surroundings that is optimised for storage stability.

A further aspect of the invention is a method of selection of conditions capable of protecting a protein in aqueous environment against heat degradation (including at ambient temperature or above), comprising selecting a pH in the range 4 to 9 at or near a value at which the protein is in a proton-exchange equilibrium state with respect to its surroundings that is optimised for storage stability.

By having a protein at a suitable pH, at or near (usually within 0.5 pH units, preferably within 0.4 pH units and more preferably within 0.2 pH units) a value at which the proton exchange equilibrium state with respect to its surroundings is optimised for storage stability, so the storage stability of the protein (either at ambient temperature (about 20°C) or at elevated temperature) can be increased, possibly substantially, compared with the storage stability of the protein in non-optimised condition (usually at pH 7).

References to optimising storage stability of a protein mean moving towards the optimum, without necessarily achieving the optimum. By bringing storage stability closer to optimum, so storage stability is improved as compared with non-optimum conditions, e.g. at pH 7.

Storage stability can generally be further enhanced by use of one or more additives including one or more species capable of exchanging protons with the protein with less extreme $pK_a$ values than those of $H_3O^+$ (which has a $pK_a$ of 13.99) and OH- (which has a $pK_a$ of -1.74). It is preferred to use one or more additives with first and second functional groups as discussed above.

Additives are suitably present in an amount such that the total concentration of additives is in the range 1 mM to 1 M, preferably 1 mM to 200 mM, most preferably 5 mM to 100 mM. Good results have been obtained with low levels of additives (around 5 mM) with certain proteins, with little benefit being obtained by using higher levels, although exceptions have been found, e.g. with catalase where higher levels of additives (100 mM) gave significantly better stability than lower levels (10 mM). In certain practical applications, especially in medical applications, it will often be desirable to use as low concentrations of additives as possible.

Discoveries underlying the present invention have a theoretical basis, as discussed herein, which have been borne out in practice. Indeed, this specification describes how a stabilising system for any protein can be determined, based on available information. Nevertheless, the benefits of the invention may also be achieved on an empirical basis, on the understanding that, for any protein in solution, and depending on the presence of other additives, there is a relationship between stabilisity and pH, and that there is a ph at which the protein has optimum stability. Based on the information presented herein, one of ordinary skill in the art can readily determine additives that are likely to be useful and then whether they in fact meet the criteria by which the invention is defined.

It appears that, prior to the present invention, the significance of proton exchange and hence pH in relation to the storage stability of proteins has not been appreciated. The present invention enables improvements to be made in the storage stability of proteins by selecting an appropriate pH, preferably in combination with one or more additives.

The invention thus enables improvements to be made in the storage stability of proteins in aqueous environment such that proteins can be stored for extended periods of time at ambient temperatures without significant loss of activity, thus avoiding the need for freezing or refrigeration. While most of the experimental examples herein use elevated temperatures (typically between 55°C and 65°C) to accelerate loss of activity, the results provide a very good indication of protein stability at lower, including ambient, temperatures.

The invention is applicable to a wide range of proteins, including polypeptides etc. as discussed above, including protein vaccines and therapeutic proteins, and including relatively small proteins/polypeptides such as glucagon (relative molecular weight 3550) and insulin (relative molecular weight 6000).

It will be appreciated that the present invention provides a general solution to the problem of stabilising proteins.

It is of course possible that the prior art has disclosed comopositions which meet the criteria described herein, without appreciating the generality of the invention. Any such disclosure, such as the prior art mentioned above, is excluded from the scope of the claims.

A further aspect of the present invention is a composition comprising a protein and one or more additives adsorbed on an adjucant. The or each additive is selected as if present in aqueous solution, as defined above (but is not necessarily water-soluble). Such a composition may be obtained by adsorption, onto an adjuvant, of the components from an aqueous composition or the invention.

Description of the Drawings

Figure 1 is a graph of % protonated/de-protonated form versus pH for the 7 protonatable amino acids, with the full line representing the protonated form and the dotted line representing the de-protonated form;

Figure 2 is a graph of the product of the normalised concentration of the protonated form [HA] and the deprotonated form [A] in arbitrary units versus pH for the 7 protonatable amino acids;

Figure 3 is a graph of proton exchange frequency (relative to the size of protein) in arbitrary units versus pH for the enzyme papain, illustrating the relative rate of proton exchanges on the surface of the papain molecule;

Figure 4 is a graph of % protonation of the amino acid side-chains of glucose oxidase and papain versus pH;

Figure 5 is a pair of graphs of proton exchange frequency (relative to size of protein) in arbitrary units versus pH for glucose oxidase and horseradish peroxidase, showing the difference between the theoretical pH optimum for protein stability calculated on the basis of the minimum overall proton exchange frequency and the actual pH optimum;

Figure 6 is a graph of concentration (in $\mu$M) versus pH for $H_3O^+$ and OH- in aqueous solution at 25°C;

Figure 7 is a graph of proton exchange frequency (relative to the size of protein) in arbitrary units P versus pH, illustrating estimation of pH optimum for protein stability based on known amino acid sequence and isoelectric point of a protein;

Figure 8 is a graph similar to Figure 3, illustrating estimation of pH optimum for stability of glucose oxidase based on known amino acid sequence and isoelectric point of the protein;

Figure 9 is a graph similar to Figure 8, for catalase;

Figure 10 is a graph similar to Figure 8, for papain;

Figure 11 is a graph similar to Figure 8, for glutamate dehydrogenase; and

Figure 12 is a graph of activity recovery of uricase activity on incubation at 60°C in a formulation containing (A) phosphate buffer (20 mM, pH 7.0), (B) TRIS/HCl buffer (20 mM, pH 7.2), (C) borate buffer (20 mM, pH 9.0), (D) purine (20 mM) + succinate (20 mM) pH 9.0, (E) TRIS (20 mM) + Serine (20 mM) pH 8.8. [uricase] = 250 $\mu$g mL$^{-1}$.

Description of Preferred Embodiments

Certain preferred features of the invention are defined in the subclaims.

It will be appreciated that this invention relates to the stability of proteins, particularly the stability of proteins in an aqueous environment, e.g. in aqueous solution, in aqueous gel form and in solid state (or other non-liquid state) where free or bound water is present, and concerns the storage stability of proteins, i.e. stability with time, including stability at ambient temperatures (about 20°C) and above. The term "protein" is used herein to encompass molecules or molecular complexes consisting of a single polypeptide, molecules or molecular complexes comprising two or more polypeptides and molecules or molecular complexes comprising one or more polypeptides together with one or more non-polypeptide moieties such as prosthetic groups, cofactors etc. The term "polypeptide" is intended to encompass polypeptides comprising covalently linked non-amino acid moieties such as glycosylated polypeptides, lipoproteins etc. In particular, the invention relates to molecules having one or more biological activities of interest which activity or activities are critically dependent on retention of a particular or native three-dimensional structure in at least a critical portion of the molecule or molecular complex. In general, the invention may be applicable to polypeptides having a molecular weight of at least 2000 (i.e. consisting of at least about 15 amino acids) where at least basic motifs of secondary or tertiary structure possibly important for protein function might be formed.

The invention is applicable to any system in which the retention of structural characteristics of a protein, in particular the secondary, tertiary and quaternary structure, and the retention of functional characteristics of the protein, in particular the enzyme activity, antigen binding, receptor binding, ligand binding or other specific binding events, are of importance.

The application of the invention reduces significantly the probability of irreversible conformational change and irreversible aggregation of a protein and consequent loss of protein activity.

The invention is applicable to stabilisation of a protein throughout its product life including isolation or expression, purification, transport and storage.

In terms of molecular size, the invention is applicable to polypeptides with a relative molecular weight of at least 2000 where at least basic motifs of secondary or tertiary structure are likely to be formed. There is no upper limit of the relative molecular weight that would limit application of the present invention.

In terms of secondary structure, the invention is applicable to proteins containing any proportion of alpha helix, beta sheet and random coil.

In terms of tertiary structure, the invention is applicable both to globular proteins and to fibrillar proteins. The invention is applicable to proteins whose tertiary structure is maintained solely by means of non-covalent interactions as well as proteins whose tertiary structure is maintained by combination of non-covalent interactions and one or more disulphide bridges.

In terms of quaternary structure, the invention is applicable to monomeric proteins as well as proteins consisting of two, three, four or more subunits. The invention is also applicable to protein conjugates.

In terms of non-protein structural components, the invention is applicable to proteins that do not contain any non-peptide components as well as glycoproteins, lipoproteins, nucleoproteins, metalloproteins and other protein containing complexes where protein represents at least 10% of the total mass. It is applicable to proteins that do not require a cofactor for their function as well as to proteins that require a coenzyme, prosthetic group or an activator for their function.

The invention is applicable to proteins dissolved freely in aqueous solutions, or present in an aqueous system as a dispersion or suspension, as well as proteins attached to solid substrates such as vaccine adjuvant or cellular membrane by means of hydrophobic, ionic or ligand exchange interactions. The invention is also applicable to proteins dissolved in aqueous gel form and proteins in solid state where water has been removed partially or fully from an aqueous solution by drying or by freeze-drying where free or bound water is still present.

The protein may be native or recombinant, glycosylated or non-glycosylated, autolytic or non-autolytic. The invention is particularly applicable to the following groups of proteins.

**Protein or peptide hormones and growth factors**

The function of protein or peptide hormones and growth factors depends on their ability to bind to a specific receptor. Such binding event is linked closely to the protein conformation. The retention of three-dimensional structure of the protein, or at least the 3-D structure of key domains, is therefore crucial for their function. The retention of structural and functional characteristics is also of paramount importance for the regulatory approval of the protein therapeutics. Examples of therapeutic protein or peptide hormones include:

Insulin (treatment of diabetes)
Glucagon (treatment of diabetes)
Human growth hormone
Gonadotropin
Human thyroid stimulation hormone (treatment of thyroid cancer)
Granulocyte colony stimulation factor (used as part of chemotherapy)

**Therapeutic enzymes**

The function of therapeutic enzymes depends directly on their molecular structure and conformation. Irreversible conformational changes and irreversible aggregation lead to inactivation of the therapeutic enzymes. The retention of the structural characteristics of the protein is also an essential pre-requisite of the regulatory approval. Examples of therapeutic protein or peptide hormones include:

Streptokinase (thrombolytic agent in treatment of ischemic stroke)
Asparaginase
Urate oxidase
Papain (tissue debridement)

**Vaccines**

The immunogenic activity of protein vaccines depends (to a large extent) on the structural integrity of the key protein antigens, especially in relation to conformational epitopes (where antibodies are required to bind disparate regions of the polypeptide chain broght together by native folding). Irreversible conformational changes and irreversible aggregation lead to inactivation of vaccines. The same considerations apply to proteins adsorbed onto particles, such as alumina particles, or other (non-particulate) surfaces when substantial regions of each protein molecule are still in full interaction with solvent water. This is of particular importance in vaccine distribution in the third world where the maintenance of the cold chain is very difficult or impossible, partly through practical or logistic limitations and partly through cost. The present invention can be applied to recombinant protein vaccines as well as attenuated viruses or whole cell vaccines, providing the key antigens consist of polypeptide chains. Examples of such vaccines include:

Hepatitis B vaccine
Malaria vaccine
Human papilloma vaccine
Meningitis A vaccine
Meningitis C vaccine
Pertussis vaccine
Polio vaccines

**Therapeutic antibodies**

The function of therapeutic antibodies is based on their specific interactions with target antigens. So, in order to maintain their function, the retention of the three-dimensional structure is essential for the duration of their shelf life. Although generally very stable at ambient temperature, due to the inherent rigid, stable structure of the immunoglobulin fold or domain, antibodies can benefit from the present invention, by further increasing their stability in storage. Examples of therapeutic antibodies that can be used, for example, in cancer therapy include:

anti-Epidermal Growth Factor Receptor (EGFR) monoclonal antibody
anti-HER2 monoclonal antibody (breast cancer therapy)
anti-CD52 monoclonal antibody (chronic lymphocytic leukaemia therapy)
anti CD20 monoclonal antibody (aggressive lymphoma therapy)

**Interferons**

Interferons are rather unstable polypeptides of therapeutic importance, that are used, for example, in multiple sclerosis therapy. Application of the present invention can increase the shelf life and cost effectiveness of interferons. Interferon beta is the main current example.

**Other therapeutic proteins**

Following are examples of other therapeutic proteins that can benefit from the application of the present invention, in terms of cost-effectiveness and improved shelf life, particularly in aqueous solution:

Erythropoietin (stimulating erythrocyte production)
Darbepoietin alpha (stimulating erythrocyte production)
Blood coaguation factors, mainly Factor VIII and Factor IX (treatment and control of haemophilia)
Immunosuppressive agents (treatment of various conditions such as asthma, allergic rhinitis or multiple sclerosis)
Human albumin
Protein C (antithrombic agent)

**Diagnostic and industrial proteins**

The retention of the structural characteristics is crucial for the function of diagnostic proteins, particularly enzymes and antibodies. In particular, in-kit reference standards of the analytes, through which the assay is calibrated and subjected to QC, must be rigorously stabilised, as any drift in their integrity will cause a resultant drift in accuracy of the whole kit. Impaired activity can lead to false results or poor performance (e.g. slow running of the procedure). Stability of the functional activity of diagnostic proteins throughout their product life is therefore of paramount importance. Manufacturers of diagnostic products are keen to find approaches and formulations that would eliminate costly lyophilisation, which causes substantial processing bottle-necks. Examples of diagnostic proteins include:

Monoclonal antibodies
Polyclonal antibodies
Antibody-enzyme conjugates
Oxidases such as glucose oxidase, galactose oxidase, cholesterol oxidase

Peroxidases
Alkaline phosphatase
Dehydrogenases such as glutamate dehydrogenase, glucose dehydrogenase Isomerases such as invertase
Hydrolases such as trypsin, or chymotrypsin

Integral assay reference standards supplied in kit form, such as hormones, growth factors, microbial proteins, metabolic proteins, soluible forms of structural proteins etc.

Examples of industrial proteins include:

Amylase
Protease
Lipase

**International reference standards of therapeutic proteins, vaccines and diagnostics**

Reference standards of therapeutic or diagnostic proteins are of great importance for standardisation of therapeutic and diagnostic procedures. The stability of reference standards is of fundamental importance. A wide range of protein-based reference standards are therefore an ideal target for the present invention.

The protein used in the invention can be maintained substabtially in its native state. For the purposes of this specification, the term "native protein" is used to describe a protein having retained tertiary structure, and distinction from proteins that have undergone a degree of unfolding or denaturation. A native protein may incorporate some chemical modification, e.g. deamidation, rather than physical modification.

The present stabilisation technology can be applied to stabilising proteins adsorbed on solid surfaces such as alumina. In some cases it is beneficial to pre-incubate the solid surface (such as alumina particles) in the stabilising formulation prior to adsorption of the protein onto the surface. This can result in greater stability of the protein.

For the particular case of immunogenic proteins, e.g. phosphoproteins, such as Hepatitis B, intended for use as vaccines, and which may therefore be used in association with an adsorbent/adjuvant such as alumina, and particularly in the case where ligand exchange may occur, phosphate is a preferred stabilising agent. It appears that phosphate may provide additional desirable properties, e.g. when formulations contain phosphate anion (> 20 mM). The reasons behind the importance of phosphate are not entirely clear, but it is believed that phosphate anion plays a role in appropriate binding of the vaccine onto alumina (see, for example, Iyer S. et al.: Vaccine 22 (2004) 1475-1479). As reported in Example 8, >95% recovery of antigenic activity was observed of the Hepatitis B vaccine on storage at 55°C for 7 weeks in the formulation based on the novel stabilisation technology in the presence of phosphate anion. Additional experiments were carried out to ensure that the vaccine remained strongly adsorbed on the alumina adjuvant and no changes occurred in the sedimentation rate of the vaccine/alumina particles. Whilst the use of phosphate is very common as a pH buffer in pharmaceutical formulations, its application in the present invention is unusual because the pH of the formulation is 5.2, i.e. a condition where the buffering capacity of phosphate is negligible.

Without wishing to be bound by theory, the present invention is based at least in part on the realisation that each exchange of proton in the energetically favourable acid -base direction is accompanied by a release of energy proportional to the difference in $pK_a$ between the amino acid side-chain and the molecule with which the proton is exchanged. The energy of such transfer will be dissipated into the surrounding environment in the form of enthalpy (heat release) and change in entropy. This can lead to a temporary change of the structural and energetic characteristics in the vicinity of the amino acid involved. Although it cannot be ruled out that such event might even result in breakage of chemical bond, the possibility of such effect is undoubtedly very small. It is however considerably more likely that such event could result in a significant (albeit temporary and localised) change in the protein conformation around the amino acid in question. In case of a number of such events occurring simultaneously in various parts of the protein molecule this could lead to the loss of the native tertiary structure ("unfolding") and subsequent irreversible loss of the protein biological function (e.g. enzyme activity).

In order to stabilise the protein molecule it is necessary to minimise the frequency and the energy of the proton exchanges at the protein surface. The following three energy-related aspects of the proton exchanges are considered in the present model:

(1) Protein stability can be enhanced by minimising the frequency of the proton exchanges at its surface.
(2) Since the free energy of all protonatable amino acid side-chains is lower in the protonated form than in the de-protonated form, protein stability can be enhanced by keeping as high a proportion of the side-chains as possible in the protonated form.
(3) Since the acid-base dynamic equilibrium of the amino acid side-chains at the surface of a protein is maintained by continuous exchanges of protons with the surrounding molecules, and since the amount of energy released during such proton exchanges depends on the difference in $pK_a$ between the species involved, it is beneficial for the protein stability to substitute the "energetic" proton exchanges (especially those involving $H_3O^+$ and $OH^-$) with "mild" ones (involving chemical species with less extreme $pK_a$ values. This can be achieved by minimising the concentration of $H_3O^+$ and $OH^-$, and Incorporating species capable of exchanging proton with less extreme $pK_a$ values.

*Frequency of proton exchange*

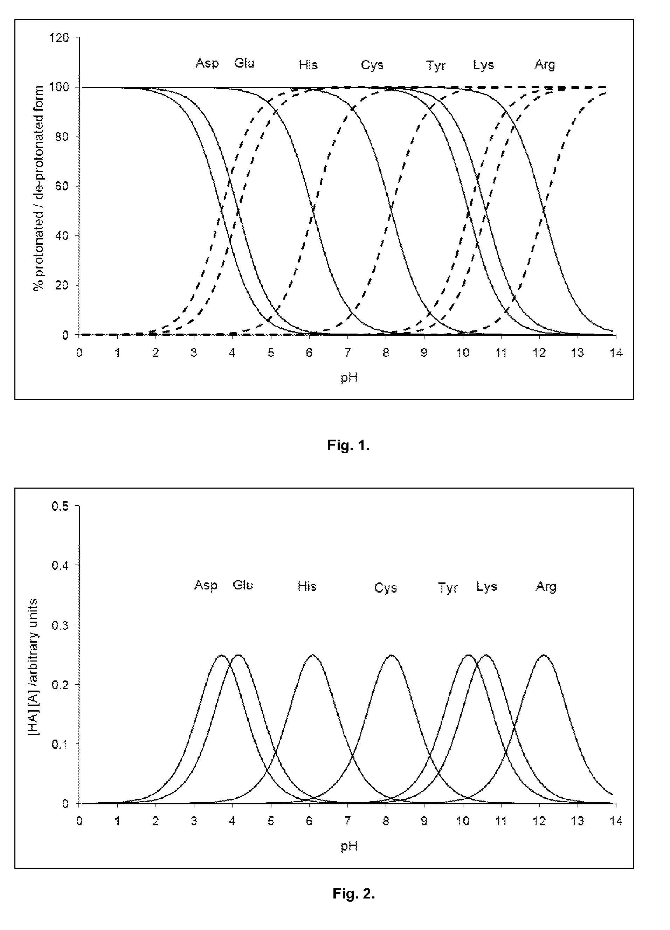

The pKa of a compound is an indication of its acid-base behaviour. The value varies with temperature, and it should be noted that the pKa value of an amino acid side-chain is not necessarily the same as that of the free amino acid (although the difference should not be too great). The pKa values of the side-chains of protein amino acids determine the relative proportion of protonated and de-protonated forms at a given pH. This is shown in Fig. 1.

The frequency (or rate) of proton exchange between an amino acid side-chain and surrounding molecules will be maximal if the pH = $pK_a$ of the amino acid. At this pH, the equilibrium is maintained so that there is an equal amount of protonated and de-protonated form of the side-chain. This results in the maximal rate of the protonation/de-protonation cycle. If the pH is remote from the $pK_a$ of the amino acid then the rate of the protonation/de-protonation cycle is reduced by the low proportion of either the protonated form (at high pH) or the de-protonated form (at low pH). For instance, in the case of glutamic acid, the maximal proton exchange frequency will occur at pH around 4.15, in the case of histidine around pH 6 etc. The relative rate of the proton exchanges of the seven amino acids is shown as a function of pH in Fig. 2. The relative rate is expressed as the product of the concentrations of the protonated (HA) and the de-protonated (A) form of the amino acid side-chain, with total non-dimensional concentration of the amino acid side-chain arbitrarily selected as 1. The value on the y-axis corresponding to the maximal rate of proton exchange is thus 0.5 times 0.5 = 0.25.

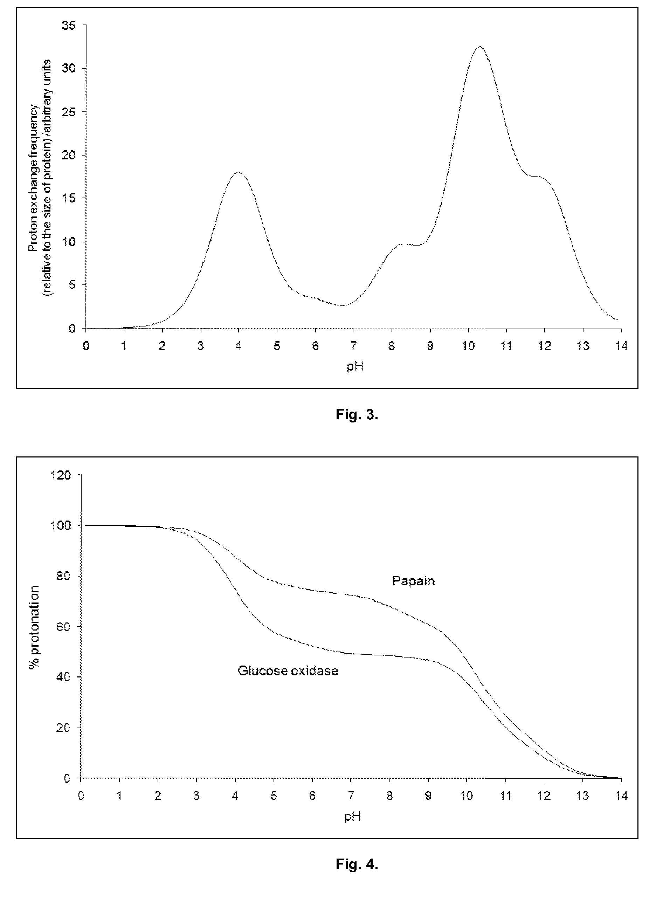

The protein stability can be enhanced by minimising the overall frequency of the proton exchanges, i.e. the sum of all proton exchange events at the protein surface. An example of the overall frequency of proton exchange frequency is shown in Fig. 3 for the protease enzyme papain. This was calculated by summing the proton exchange frequencies of the individual amino acids (as shown in Fig. 2) while the relative contribution of each amino acid was determined by its the relative abundance in the papain sequence. The pH optimum for papain stability was found experimentally to be between 5.8 and 6.2. This corresponds approximately to the minimum of the proton exchange rate (Fig. 3).

However, the relative frequency of the proton exchange is only one of the contributing factors to the protein stability/instability and has to be considered in the context of other contributing factors (see below). This means that, whilst the frequency of the proton exchange is a good indication of the pH optimum, the highest stability is not necessarily attained precisely in the minimum of the proton exchange frequency profile. This is demonstrated in some of the Examples, below.

*Minimising free (Gibbs) energy of the side-chains*

The Gibbs free energy of all protonatable amino acid side-chains is lower in the protonated form than in the de-protonated form. This is because the free electron pairs (onto which the proton binds) are lower in energy in the presence of the proton compared with its absence. Binding of protons onto the amino acid side-chains thus decreases overall free energy of the entire protein. The protein stability can therefore be enhanced by maximising the protonation of the amino acids on its surface.

The percentage of overall protonation at the surface of two model proteins (glucose oxidase and papain) as a function of pH is shown in Fig. 4. This was calculated by summing the proportion of the protonated form of all protonatable amino acids in the sequence of the two proteins. Whilst glucose oxidase is an example of a protein with low isoelectric point (pl = approx. 4.4), papain is an enzyme with very high isolelectric point (pl = approx. 8.7). This is reflected in the protonation profile as shown in Fig. 4. The percentage of side-chain protonation at neutral pH (-7) is considerably higher in the case of papain than in the case of glucose oxidase. There will therefore be a greater tendency to push the pH optimum towards lower pH in the case of glucose oxidase than in the case of papain.

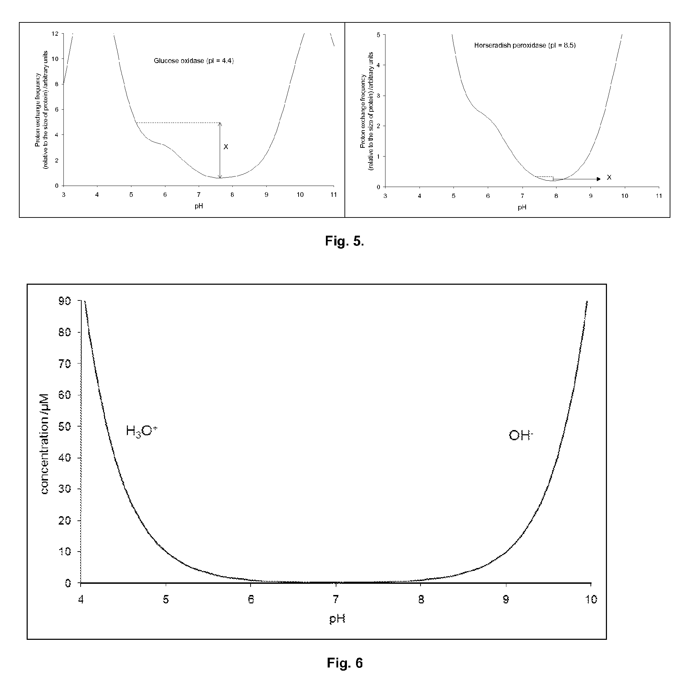

It was found experimentally that the actual pH optimum for the enzyme stability is always at least slightly (or more considerably) away towards low pH from the theoretical pH optimum calculated on the basis of the proton exchange frequency. The degree of protonation is apparently the crucial parameter that determines how far towards the low pH the optimum is shifted from the theoretical pH optimum calculated on the basis of the proton exchange frequency. Since the protonation degree is closely linked to the pl of the protein this effect can be summarised as follows: whilst proteins with high pl will only have a small tendency to shift their actual pH optimum towards low pH the proteins with low pl will have a greater tendency to shift the pH optimum in this direction. The comparison is best demonstrated with a practical example of two proteins with very different pl values, i.e. glucose oxidase and horseradish peroxidase (HRP). As shown in Fig. 5, the theoretical pH optimum calculated on the basis of the overall proton exchange frequency is approximately 7.5 in the case of glucose oxidase and 7.9 in the case of HRP. The actual pH optimum of both of these proteins is lower than the theoretical one. This means that the proteins are "sacrificing" a small portion of the stability achieved by the minimised proton exchange frequency in order to achieve greater protonation of its surface. The portion that the enzyme is willing to sacrifice (as demonstrated in Fig 5) has been repeatedly shown to be inversely proportional to the pI of the protein. Thus, the tendency of glucose oxidase to push its pH optimum lower is much greater than that of HRP (Fig. 5).

*Minimising the energy released during the proton exchanges*

The amount of energy released during the proton exchanges depends on the difference in $pK_a$ between the species involved. It is therefore beneficial for the stability of the protein to minimise the impact of continuous interactions with compounds of extreme $pK_a$ (especially those involving $H_3O^+$ and $OH^-$) and ensure that these are replaced by interactions with compounds with less extreme $pK_a$ values.

The dominant species with "extreme $pK_a$ values" in aqueous solutions are $H_3O^+$ and OH-The more extreme is the $pK_a$ value of a species, the less likely it is that this species occurs in the "high energy" form (i.e. protonated form in the case of a strong acid or the deprotonated form in the case of strong base). It can be said that, the more harmful the species is for the protein, the less likely it is to occur in the aqueous solution, this being a useful feedback mechanism protecting the protein. Such feedback mechanism also applies to $H_3O^+$ and $OH^-$. However, these two species are derived from water (by protonation or de-protonation) whose concentration in aqueous solutions is extremely high (-55.5 M) which, in turn, increases the relative abundance of these "high energy" species. Therefore, given the extreme $pK_a$ of $H_3O^+$ and OH- the concentration of these species will always be very high (e.g. three orders of magnitude higher than concentration of other species of comparable $pK_a$ derived from an additive present at 55.5 mM concentration).

It should be noted that there are other species with extreme $pK_a$ values that can be used relatively commonly in connection with enzyme formulations. One such example is a sodium cation ($Na^+$). The $pK_a$ of $Na^+$ at 25°C is 14.8 (Handbook of Chemistry and Physics, 1998). This means that the proton exchanges between the $Na^+$ and a protein molecule can be quite "energetic". Due to the above mentioned feedback protective mechanism the concentration of the "high energy form" of $Na^+$ will be considerably lower at any given pH than that of equally "energetic" OH- (i.e. a species with very similar $pK_a$ as the sodium cation). Nevertheless, a de-stabilising effect of $Na^+$ on model proteins was still observed in a number of experiments (especially if higher concentrations of $Na^+$ salts were used). In contrast, no de-stabilising effect was observed for the ammonium cation ($NH_4^+$), a species with considerably less extreme $pK_a$ (approximately 9).

Although the destructive "energetic" proton exchanges between the amino acid side-chains and either $H_3O^+$ or OH- cannot be completely avoided, it is possible to decrease considerably their thermodynamic probability and thus make the enzyme more stable. This can be achieved by adjusting the pH to such a value where the destructive ability of "energetic" proton exchanges is minimal due to the protonation status of the protein amino acid side-chains and due to concentrations of $H_3O^+$ and $OH^-$; and by incorporating a compound or ideally a combination of compounds capable of "mild" exchanges of protons with the amino acid side-chains on the surface of the protein molecule. Those "mild" exchanges will be competing with the destructive "energetic" exchanges caused by $H_3O^+$ or OH- and reduce their impact.

The concentrations of $H_3O^+$ and OH- in aqueous solutions at 25°C of given pH are shown in Fig. 6. There would be some logic in asserting that the optimum pH for protein stability must be 7, because this is the pH where the total concentration of $H_3O^+$ and OH- is at its minimum (200 nM). For comparison, the total concentration of $H_3O^+$ and OH- at pH 6 or 8 is 1.1 $\mu$M, at pH 5 or 9 it is 10.01 $\mu$M and so on. Nevertheless, this would only be the case if the concentration of $H_3O^+$ and OH- was the only parameter determining the probability of destructive proton exchanges. The probability also depends on the protonation status of the protein at the given pH, as discussed above. Furthermore, it depends on the overall amino acid composition of the protein, which determines whether the protein is overall more susceptible to the $H_3O^+$ attack or the OH- attack. The pH optimum can thus be quite remote from 7.

The energy impact of the proton exchanges between the amino acid side-chains and $H_3O^+$ or OH- is indicated in Table 1. The impact is expressed as the difference in $pK_a$ between the colliding species (the difference in $pK_a$ being proportional to the energy released during the collision).

**Table 1**

| Amino acid | $pK_a$ | $pK_a$ - $pK_{H3O+}$ | $pK_{OH-}$ - $pK_a$ |
|---|---|---|---|
| Aspartic acid | 3.71 | 5.45 | 10.28 |
| Glutamic acid | 4.15 | 5.89 | 9.84 |
| Histidine | 6.04 | 7.78 | 7.95 |
| Cysteine | 8.14 | 9.88 | 5.85 |

(continued)

| Amino acid | $pK_a$ | $pK_a$ - $pK_{H3O+}$ | $pK_{OH-}$ - $pK_a$ |
|---|---|---|---|
| Tyrosine | 10.10 | 11.84 | 3.89 |
| Lysine | 10.67 | 12.41 | 3.32 |
| Arginine | 12.1 | 13.84 | 1.89 |

Whilst in the case of the more acidic amino acids (aspartic acid, glutamic acid and histidine) it is the interaction with OH⁻ that results in more energy release, in the case of the more alkaline amino acids it is the interaction with the $H_3O^+$ that is more de-stabilising.

Because of the tendency of the protein to keep as many amino acid side-chains as possible in the low energy protonated form, it can be assumed that the three most alkaline side-chains (tyrosine, lysine and arginine - all with $pK_a$ > 10) will be virtually fully protonated in the optimum conditions and their engagement in the proton exchange will therefore be minimal. This assumption is in accord with experimental results: the pH optima of the model proteins tested ranged between 4.8 - 8.0. Even at pH 8.0 tyrosine, lysine and arginine are almost fully protonated. It is therefore the relative abundance of cysteine, histidine, glutamic acid and aspartic acid that determines the susceptibility of the protein to $H_3O^+$ or OH⁻ and thus the optimum conditions for the protein stability.

There will be a tendency of the proteins with low pl to push their pH optimum towards lower values. This is because the low pl proteins contain a higher proportion of the most acidic amino acids (aspartic acid and glutamic acid), which are particularly susceptible to OH⁻ attack (see Table 1).

The pH adjustment is not the only measure that can be taken to improve the protein stability. The stability can be further improved by adding compounds that can engage in "mild" proton exchanges with the amino acid side-chains of the protein and thus reduce further the probability of the destructive "energetic" proton exchanges with $H_3O^+$ or OH⁻.

It was found experimentally that the best results can be achieved if two acid-base functional groups are incorporated. Preferably, the first functional group has a $pK_a$ at least one unit higher than the pH optimum and is positively charged at the pH optimum. This means that the charge of the group switches from neutral to positive while accepting the proton (e.g. amino group, purine, TRIS etc.). The second functional group preferably has a $pK_a$ at least one unit lower than the pH optimum and is negatively charged at the pH optimum. This means that the charge of the group switches from negative to neutral while accepting the proton (e.g. carboxylic group).

A small amount of buffer can be used alongside the additives in order to maintain the optimal pH. The incorporation of the additives can affect slightly the pH optimum for the protein stability (within approximately 0.5 pH unit depending on the nature and quantity of the additives).

It was also found that a relative excess of the first group (i.e. the positively charged at the pH optimum) is beneficial for the protein stability. This can be speculatively explained by forming ionic bonds with the negatively charged side-chains of aspartic acids and glutamic acids (these side-chains will be largely unprotonated at the pH optimum in the case of practically every enzyme - pH optimum would have to be far below 4 in order for these side-chains to become largely protonated). Such ionic bonds might lower the Gibbs energy of the free electron pair of these side-chains - just as binding of a proton does.

An algorithm has been derived, to allow a good prediction of the pH optimum based on the known amino acid sequence of the protein and its isoelectric point. Whilst the prediction can be achieved using the total amino acid sequence, it is recommended to only take into account the amino acid side-chains that are accessible at the surface of the protein. Both the total amino acid sequences and the accessibility of the individual amino acids in the protein structure can be found using the Protein Data Bank information database (http://www.rcsb.org/pdb). For the amino acid accessibility estimation it is necessary to download the DEEP VIEW protein software (available at http://www.expasy.org/spdbv)

The algorithm consists of three steps as follows

**Step 1:** (this can be done easily in MS-Excel)

Calculate the proton exchange frequency function (P) and plot against pH:

$$P = \frac{100000}{M} \times \sum_1^7 (N \times [HA] \times [A])$$

where

EP 2 264 161 A1

$$[HA] = \cfrac{1}{1 + \cfrac{10^{-pKa}}{10^{-pH}}} \qquad\qquad [A] = \cfrac{10^{-pKa}}{10^{-pKa} + 10^{-pH}}$$

M is the relative molecular weight of the protein unit

N is the number of the given amino acid side-chains in the protein unit (1 = aspartic acid, 2 + glutamic acid, 3 = histidine etc.)

[HA] is the proportion of the protonated form of the amino acid at the given pH. 0 < [HA] < 1.

[A] is the proportion of the de-protonated form of the amino acid at the given pH. 0 < [A] < 1.

If plotted against pH the proton exchange frequency profile can be obtained (like the one shown in Fig. 3 for papain).

**Step 2:** Calculate the magnitude of the pH shift (denoted here as X) as follows:

$$X = A \times pI + B$$

where A = -1.192 and B = 10.587 if the total amino acid composition is used;

And A = -0.931 and B = 8.430 if only the amino acids accessible at the protein surface is used.

Find minimum of the function obtained in step 1 in the pH interval 4-9. Add the calculated pH shift value to the minimum to obtain value Y:

$$Y = P_{minimum} + X$$

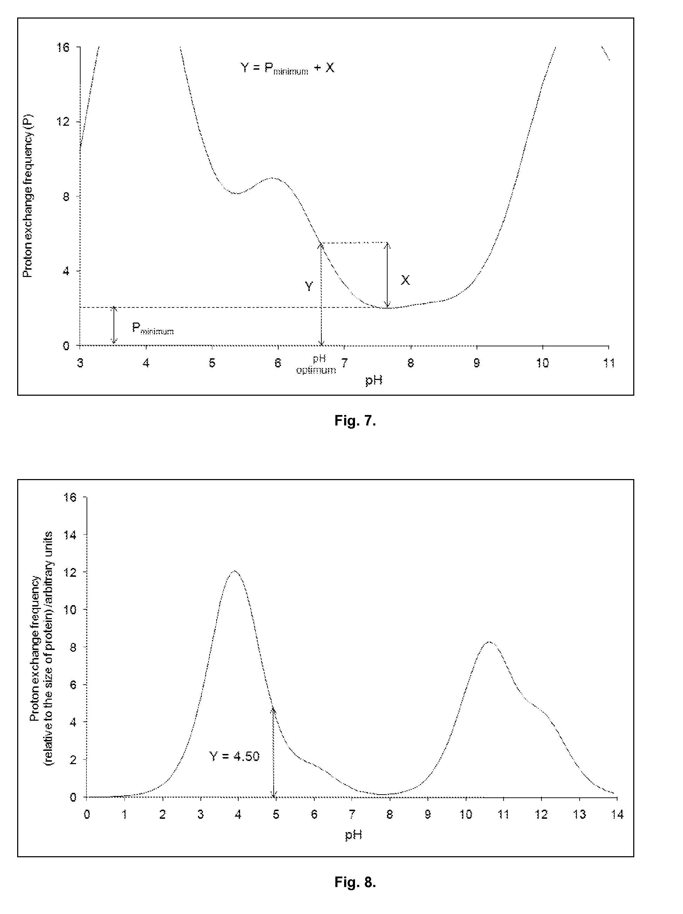

Read the pH that corresponds to the value Y in the P vs. pH graph. There will always be two pH values that correspond to the value Y in the P vs. pH graph (one below the $P_{minimum}$ and one above the $P_{minimum}$. It is important that the value is read below the $P_{minimum}$ (i.e. toward the lower pH values). This is the estimated pH optimum for the stability of the enzyme. The relation between parameters X, Y and $P_{minimum}$ is demonstrated in Fig. 7.

The values of A and B were calculated based on the results of a series of experiments using four model enzymes (glucose oxidase, catalase, lactoperoxidase and papain). These values were subsequently used to predict pH optimum of two further enzymes (horseradish peroxidase and glutamate dehydrogenase), and experiments showed the predictions had a very good precision.

**Step 3:**

Once the pH optimum is selected, one or more additives that contain one or more of the following functional groups may be chosen.

Functional group 1:

The first functional group has a $pK_a$ higher than the pH optimum for the protein stability (as estimated in steps 1 and 2) and is positively charged at the pH optimum. This means that the charge of the group switches from neutral to positive while accepting the proton.

The $pK_a$ of the functional group must be higher than the pH optimum for the protein stability. Preferably, it should be within 5 pH units above the pH optimum. More preferably, it should be within 0.5 - 4 pH units above the pH optimum. Most preferably, it should be within 1 - 3 pH units above the pH optimum for the enzyme.

Functional group 2:

The second functional group has a $pK_a$ at least one unit lower than the pH optimum for the protein stability (as estimated in steps 1 and 2) and is negatively charged at the pH optimum. This means that the charge of the group switches from negative to neutral while accepting the proton (e.g. carboxylic group).

12

The pK$_a$ of the functional group must be lower than the pH optimum for the protein stability. Preferably, it should be within 5 pH units below the pH optimum. More preferably, it should be within 0.5 - 4 pH units above the pH optimum. Most preferably, it should be within 1 - 3 pH units below the pH optimum for the enzyme.

The formulation can only contain the first functional group (i.e. the positively charged one). Preferably, the formulation contains both the first and the second functional group.

The two functional groups can be contained within one compound (e.g. an amino acid). Preferably, the two functional groups are located on more than one additive (e.g. one additive contains the first, negatively charged group, and the other additive contains the second, positively charged group). There is no limit to the number of each of the two types of functional groups added to the formulation.

In some cases it may be beneficial to incorporate the positively charged functional group in excess relative to the amount of the negatively charged group.

The buffering ability of the additives can be sufficient in those cases where their pK$_a$ is only about 1 pH unit from the pH optimum. If the pK$_a$ is further away their buffering ability may be reduced. A small concentration of buffer with pK$_a$ close to the pH optimum can then be used in order to maintain the required pH.

Some examples of classes of groups for each of the two functional groups that can be usefully incorporated as the stabilising additives is shown in Table 2.

**Table 2**

| Functional group 1 | Functional group 2 |
|---|---|
| $\alpha$-Amino group of amino acids | $\alpha$-Carboxylic group of amino acids |
| Side-chain amino group of amino acids | Side-chain carboxylic group of amino acids |
| Purine (and purine based compounds) | Carboxylic group of organic acids such as: |
| Primary amines | Lactic acid |
| Secondary amines | Succinic acid |
| Tertiary amines | |
| Quarternary amines such as TRIS | Inorganic acids |

A list of specific examples of possible additive components, with pK$_a$ values, is given in Table 3.

**Table 3**

| Amino Acids | pK$_a$ of functional group 1 | pK$_a$ of functional group 2 |
|---|---|---|
| Glycine | 9.8 | 2.4 |
| Alanine | 9.9 | 2.4 |
| Valine | 9.7 | 2.2 |
| Leucine | 9.7 | 2.3 |
| Isoleucine | 9.8 | 2.3 |
| Serine | 9.2 | 2.2 |
| Threonine | 9.1 | 2.1 |
| Cysteine | 10.8, 8.3 | 1.9 |
| Methionine | 9.3 | 2.1 |
| Aspartic acid | 9.9 | 2.0, 3.7 |
| Asparagine | 8.8 | 2.1 |
| Glutamic acid | 9.5 | 2.1, 4.1 |
| Glutamine | 9.1 | 2.2 |
| Arginine | 9.0 | 1.8 |

(continued)

| Amino Acids | $pK_a$ of functional group 1 | $pK_a$ of functional group 2 |
|---|---|---|
| Lysine | 9.2 | 2.2 |
| Histidine | 9.2, 6.0 | 1.8 |
| Phenylalanine | 9.2 | 2.2 |
| Tyrosine | 9.1 | 2.2 |
| Tryptophan | 9.4 | 2.4 |
| Proline | 10.6 | 2.0 |
| Ornithine | 8.69 | 1.7 |
| Citrulline | 9.69 | 2.4 |
| & various peptides consisting of the above | | |
| amino acids | | |
| **Other carboxylic acids** | | |
| Formic acid | | 3.8 |
| Glyoxylic acid | | 3.2 |
| Oxalic acid | | 1.3, 4.2 |
| Acetic acid | | 4.8 |
| Glycolic acid | | 3.8 |
| Pyruvic acid | | 2.4 |
| Malonic acid | | 2.8, 5.7 |
| Lactic acid | | 3.8 |
| Glyceric acid | | 3.5 |
| Fumaric acid | | 3.0, 4.4 |
| Succinic acid | | 4.2, 5.6 |
| Malic acid | | 3.4, 5.1 |
| $\alpha$-Tartaric acid | | 3.0, 4.3 |
| Glutaric acid | | 4.3, 5.4 |
| Ascorbic acid | | 4.1 |
| Adipic acid | | 4.4 |
| Gallic acid | | 4.4 |
| **Amines** | | |
| Trimethylamine | 9.8 | |
| 1,2-Propanediamine | 6.66, 9.8 | |
| 1,3-Propanediamine | 9.0, 10.3 | |
| 1,2,3-Triaminopropane | 9.5, 7.9 | |
| Pentylamine | 10.6 | |
| Tris(hydroxymethyl)aminomethane | 8.1 | |

(continued)

| Amines | | |
|---|---|---|
| Benzylamine | 9.3 | |
| Phenyl ethylamine | 9.8 | |
| Tyramine | 9.7 | |
| Tryptamine | 10.2 | |
| Hydroxytryptamine | 9.8 | |
| **Imines** | | |
| Ethyleneimine | 8.01 | |
| **Other compounds** | | |
| Purine | 8.96 | |
| 8-hydroxypurine | 8.26 | |
| Ammonium ion | 9.25 | |
| Quinoline | 4.9 | |
| 1-Isoquinolineamine | 7.6 | |
| 1-Methylimidazol | 7.0 | |
| Allantoin | 8.9 | |
| Veronal | 7.4 | |
| 2-Ethylbenzimidazole | 6.2 | |
| Brucine | 8.3 | |
| Uracil | 9.5 | |
| 2,4,6-Trimethylpyridine | 7.5 | |

The materials listed above are given for the purpose of illustration only. It will of course be understood by one of ordinary skill in the art that aspects specific to particular proteins have to be taken into account. For instance, it is important to ensure that the additives selected do not inhibit the protein activity. It is also important to ensure that the compounds used to improve heat stability of proteins are themselves stable under the conditions employed.

The invention can be combined with other well established approaches to protein stability. For example, a protease inhibitor can be incorporated in the formulation to ensure that the protein is not slowly digested by protease activity present in the sample.

Another additive that may be used is a polyalcohol, e.g. at a concentration of at least 0.5%, and typically up to 5% (w/w). Examples of such compounds are saccharides such as inositol, lactitol, mannitol, xylitol and trehalose.

The ionic strength of the protein formulation stabilised by application of the present invention can be adjusted to meet the requirement for the intended use of the formulation (e.g. isotonic formulation for therapeutic use). Importantly, experiments showed repeatedly that in principle the stability of proteins at room temperature mirrors that at higher temperature, the rate of activity decline being many orders of magnitude slower at room temperature compared with that at increased temperature (e.g. 60°C).

Examples

The following Examples illustrate the invention.

Materials

Ammonium sulphate (Fisher, Code A/6480/60)

BANA-Na-Benzoyl-DL-arginine-β-naphthylamide hydrochloride (Sigma, Code B4750)
Catalase (from bovine liver, Sigma C9322, 2380 U /mg solid)
L-Cysteine (Fluka, Code 449808/1)
Citric acid (Fisher, Code C/6200/53)
Deionised water (conductivity < 10 $\mu$S cm$^{-1}$; either analytical reagent grade, Fisher or Sanyo Fistreem MultiPure)
Disodium hydrogen orthophosphate (Fisher, Code S/4520/53)
DMAC - 4-(Dimethylamino)cinnamaldehyde (Sigma, Code D4506)
DMSO - Dimethyl sulfoxide (Sigma-Aldrich Code154938-500)
EDTA di-sodium salt (Fisher, Code BPE120-500)
Glucose (Fisher, Code G050061)
Glucose Oxidase (Biocatalysts G575P ~150 U /mg solid)
Glutamate dehydrogenase from bovine liver (BioChemika Code 49392)
Horseradish peroxidase (Biocatalysts Code P558P)
Hydrochloric acid (Fisher, Code J/4310/17)
Hydrogen peroxide (Sigma H1009)
D,L-Lactic acid (Fluka, Code 1077141)
Lactoperoxidase (from bovine milk, DMV International: 1,050 units mg$^{-1}$ by ABTS method pH 5.0)
Lysine (Sigma, Code L5501)
Methanol (Fisher, Code M/3950/17)
NADH-β-Nicotinamide adenine di-nucleotide reduced form, di-sodium salt (Sigma N-8129)
2-Oxoglutaric acid, di-sodium salt, dihydrated (Fluka, Code 75892)
Papain (700TU ex Biocatalysts)
PBS - Phosphate buffered saline (Sigma D1408)
Potassium iodide (Fisher, Code 5880/53)
Sodium hydroxide (Fisher, Code J/7800/15)
Sodium dihydrogen orthophosphate (Fisher, Code S/3760/60)
Starch (Acros Organics, Code 177132500)
TMB - Tetramethylbenzidine (Sigma T-2885)
TRIS base - Tris(hydroxymethyl)aminomethane (Fisher Bioreagents, CodeBPE152-1)

Unless stated otherwise, phosphate buffers of given concentration and pH ($\underline{X}$ mM, pH $\underline{Y}$) used in this work were prepared by mixing disodium hydrogen orthophosphate ($\underline{X}$ mM) with sodium dihydrogen orthophosphate ($\underline{X}$ mM) to achieve the required pH $\underline{Y}$.

Unless stated otherwise, citrate/phosphate buffers of given concentration and pH ($\underline{X}$ mM, pH $\underline{Y}$) used in this work were prepared by mixing di-sodium hydrogen orthophosphate ($\underline{X}$ mM) with citric acid ($\underline{X}$ mM) to achieve the required pH $\underline{Y}$.

Overall Experimental Plan

In each example, an aqueous solution of a given enzyme was prepared with selected additives in an Eppendorf tube. Unless stated otherwise, the concentrations of enzymes used were as follows:

| | |
|---|---|
| Glucose oxidase: | 350 $\mu$g /mL |
| Catalase: | 100 $\mu$g /mL |
| Lactoperoxidase: | 100 $\mu$g /mL |
| Horseradish peroxidase: | 10 $\mu$g /mL |
| Glutamate dehydrogenase: | 150 $\mu$g /mL |
| Papain | 100 $\mu$g /mL |

Each solution was assayed for enzyme activity. The Eppendorf tubes were then immersed in the water bath (set at increased temperature between 55 - 65°C as specified in each example) for a given period of time. The solution was then assayed for remaining enzyme activity. Temperatures above ambient were used so as to be more demanding than work at ambient, and to provide more quickly an indication of protein stability.

The Examples may include information on the particular enzyme, procedure to select optimum conditions for stability (using the algorithm described above) based on surface amino acids, and practical examples demonstrating the stability of the enzyme at increased temperature. All amino acid sequences were obtained from the publicly available Protein Data Bank (http://www.rcsb.org/pdb).

Example 1 - Glucose oxidase (from Penicillium sp.)

Glucose oxidase (from Penicillium sp.) consists of two identical subunits ($M_r$ of each approx. 80,000). The isoelectric point of glucose oxidase as approximately 4.3. The abundance of the acid-base amino acids in each subunit is shown in Table 4.

**Table 4**

| Amino acid | Total number of side-chains in the subunit | Number of side-chains exposed at the subunit surface |
|---|---|---|
| Aspartic acid (D) | 36 | 23 |
| Glutamic acid (E) | 23 | 17 |
| Histidine (H) | 8 | 3 |
| Cysteine (C) | 1 (3)* | 0 |
| Tyrosine (Y) | 19 | 6 |
| Lysine (K) | 27 | 21 |
| Arginine (R) | 17 | 11 |
| * The first number indicates the number of cysteines that are not engaged in disulphide bond. The number in brackets indicates the total number of cysteines in the subunit. | | |

The proton exchange frequency profile for glucose oxidase is shown in Fig. 8. The pH optimisation algorithm gives the following results for glucose oxidase:

$$P_{minimum} = 0.17 \text{ (at pH 7.7)}$$

$$X = 4.33$$

$$Y = 4.50$$

Estimated pH optimum = 5.0

Glucose oxidase solutions, both fresh and after incubation at increased temperature, were assayed for glucose oxidase activity. This was performed according to the following procedure:

50 $\mu$L of the solution was added to 50 mL of deionised water. The following reagents were then added:

- 10 mL of reagent mix (5.5 parts of 0.1 M sodium dihydrogen orthophosphate, pH 6 + 4 parts 2% w/w starch + 0.5 part of 1mg/mL lactoperoxidase enzyme);
- 5 mL of 100 mM potassium iodide and
- 5 mL of 20% w/w glucose solution.

These were mixed together quickly. Time = 0 was counted from the addition of the glucose. After 5 min, 1 ml of 5 M aq. hydrochloric acid was added to stop the reaction. The absorbance was then read at 630 nm using a Unicam UV-visible spectrophotometer (Type: Helios gamma). If the colour intensity was too great to allow an accurate reading, the sample was diluted with a defined volume of deionised water to bring the colour back on scale. The results were expressed as percentage recovery, by reference to the absorbance measured in the fresh samples (i.e. prior to incubation at increased temperature).

Example 1.1: Poor activity recovery on incubation of glucose oxidase outside of the calculated pH optimum in the absence of beneficial additives

The effect of 50 mM phosphate buffer ($pK_a$ = 7.2) on the stability of glucose oxidase at 60°C was investigated in the pH range 6.0 to 8.0. The phosphate buffer was prepared by mixing di-sodium hydrogen orthophosphate (50 mM) and sodium dihydrogen orthophosphate (50 mM) to achieve the required pH. There was no measurable activity following incubation of glucose oxidase at 60°C for 15 minutes in the presence of phosphate at pH 7.0, 7.5 or 8.0. Some activity was measurable at 15 min at pH 6.5 and 6.0, but this fell to zero in 60 minutes. Better recovery of glucose oxidase activity was observed in deionised water. Nevertheless, even in this case the activity fell to zero after 180 min. The pH of glucose oxidase solution in DI water was approximately 6. This is the result of the buffering ability of the enzyme itself and of the impurities in the enzyme preparation. The recovery of the activity was poor because the pH was outside of the calculated pH optimum and the formulation did not contain the beneficial additives.

Example 1.2: Poor activity recovery on incubation of glucose oxidase outside of the calculated pH optimum in the presence of beneficial additives

The effect of 50 mM TRIS buffer ($pK_a$ = 8.3) in the presence of lactic acid on the stability of glucose oxidase at 60°C was investigated in the pH range 7.5 to 9.0. Tris buffer was prepared by mixing Tris base (50 mM) and lactic acid (50 mM) to achieve the required pH. There was no measurable activity following incubation of glucose oxidase at 60°C for 15 minutes in the presence of TRIS/lactate at pH 7.0, 7.5, 8.0, 8.5 or 9.0. The recovery of the activity was poor in spite of the presence of beneficial additives. This is because the pH was far out from the calculated pH optimum.

Example 1.3: Good activity recovery on incubation of glucose oxidase near the calculated pH optimum, but in the absence of beneficial additives

The effect of 50 mM citrate buffer ($pK_{a1}$ = 3.2, $pK_{a2}$ = 4.8, $pK_{a3}$ = 6.4) on the stability of glucose oxidase at 60°C was investigated in the pH range 4.4 to 5.4. Citrate buffer was prepared by mixing citric acid (50 mM) with sodium hydroxide (5 M) to achieve the required pH. The decline of the enzyme activity was slowest at pH 4.8. Over 15% of the original activity was still measurable after 22 h incubation at 60°C. The decline of the enzyme activity was only slightly lower using the same formulations at pH 4.6 and 5.0. Nevertheless, using the same formulation with pH adjusted to >0.4 unit away from the optimum resulted in considerably faster decline of the enzyme activity.

Example 1.4: Very good activity recovery on incubation of glucose oxidase near the calculated pH optimum in the presence of beneficial additives

Incorporation of glutamate and histidine into the glucose oxidase formulation adjusted to the near-optimum pH resulted in the best preservation of glucose oxidase activity on incubation at 60°C. The background solution was prepared by mixing glutamic acid (50 mM) and histidine (50 mM) and adjusting pH to the required value using either hydrochloric acid (5 M) or sodium hydroxide. Over 50 % of the original activity was still measurable after 22 h incubation at 60°C. The formulation contained a positively charged protonatable group with $pK_a$ above the optimum pH (i.e. amino group of the two amino acids - $pK_a$ around 9) and a negatively charged protonatable group with $pK_a$ below the optimum pH (i.e. carboxylic groups of the two amino acids - $pK_a$ between 3 - 4.2).

Example 2 - Catalase (from bovine liver)

Catalase (from bovine liver) consists of four identical subunits ($M_r$ of each approx. 65,000). The isoelectric point of catalase is approximately 5.7. The abundance of the acid-base amino acids in each subunit is shown in Table 5

**Table 5**

| Amino acid | Total number of side-chains in the subunit | Number of side-chains exposed at the subunit surface |
|---|---|---|
| Aspartic acid (D) | 39 | 19 |
| Glutamic acid (E) | 25 | 17 |
| Histidine (H) | 21 | 14 |
| Cysteine (C) | 4 | 0 |

(continued)

| Amino acid | Total number of side-chains in the subunit | Number of side-chains exposed at the subunit surface |
|---|---|---|
| Tyrosine (Y) | 20 | 7 |
| Lysine (K) | 27 | 17 |
| Arginine (R) | 31 | 21 |

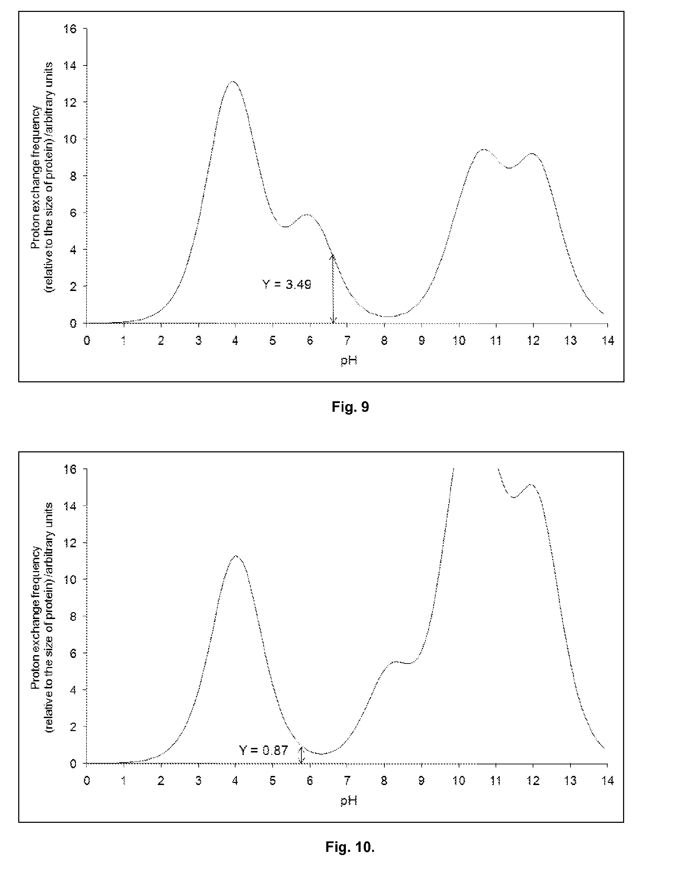

The proton exchange frequency profile for catalase is shown in Fig. 9. The pH optimisation algorithm gives the following results for catalase:

$$P_{minimum} = 0.37 \text{ (at pH 8.0)}$$

$$X = 3.12$$

$$Y = 3.49$$

Estimated pH optimum = 6.6

Catalase solutions, both fresh and after incubation at increased temperature, were assayed for catalase activity. This was performed according to the following procedure:

2 mL of hydrogen peroxide (30 mM in water) was added to 18 mL of PBS in a 125 mL polypropylene pot. 100 $\mu$L of the catalase sample was added and mixed. The resulting mixture was incubated at room temperature precisely for 30 min. In the meantime, the following reagents were mixed in a plastic cuvette for spectrophotometric measurements:

- 2.73 mL of citrate/phosphate buffer (0.1 M, pH 5.0)
- 100 $\mu$L of TMB (3 mg /mL, dissolved in DMSO)
- 100 $\mu$L of lactoperoxidase

Following the 30 min incubation period, 70 $\mu$L of the catalase containing mixture was added to the cuvette and absorbance was read in approximately 30 s. The results were expressed as percentage recovery, by reference to the absorbance measured in the fresh samples (i.e. prior to incubation at increased temperature).

Example 2.1: Poor activity recovery on incubation of catalase outside of the calculated pH optimum both in the presence and in the absence of beneficial additives

The effect of 50 mM citrate buffer (p$K_{a1}$ = 4.8, p$K_{a2}$ = 6.4) and of TRIS buffer on the stability of catalase at 55°C was investigated outside of the estimated pH optimum for catalase. Citrate buffer was prepared by mixing citric acid (50 mM) with sodium hydroxide (5 M) to achieve the required pH. TRIS buffer was prepared by mixing Tris base (50 mM) with hydrochloric acid (5 M) to achieve required pH. There was virtually no measurable activity following incubation of catalase at 55°C for 60 minutes in the presence of citrate buffer at pH 4.5 or in the presence of TRIS buffer at pH 8.2. The recovery of the activity was poor because the pH was outside of the calculated pH optimum (in spite of the presence of the "beneficial additive" in the case of TRIS).

Example 2.2: Good activity recovery on incubation of catalase near the calculated pH optimum, but in the absence of beneficial additives

The effect of 50 mM citrate buffer (p$K_{a1}$ = 4.8, p$K_{a2}$ = 6.4) on the stability of glucose oxidase at 60°C was investigated in the pH range 5.2 to 7.2. Citrate buffer was prepared by mixing citric acid (50 mM) with sodium hydroxide (5 M) to achieve the required pH. The decline of the enzyme activity was slowest at pH 6.4. Over 20% of the original activity was still measurable after 22 h incubation at 55°C. The decline of the enzyme activity was only slightly more rapid using the same formulations at pH 6.8 and 6.0. Nevertheless, using the same formulation with pH adjusted to >0.4 unit away from the optimum resulted in considerably faster decline of the enzyme activity.

Example 2.3: Very good activity recovery on incubation of catalase near the calculated pH optimum in the presence of beneficial additives

Incorporation of histidine (50 mM) into the catalase formulation adjusted to the near-optimum pH resulted in the best preservation of glucose oxidase activity on incubation at 55°C. Over 40% of the original activity was still measurable after 22 h incubation at 55°C. The formulation contained a positively charged protonatable group with $pK_a$ above the optimum pH (i.e. amino group of histidine - $pK_a$ around 9) and a negatively charged protonatable group with $pK_a$ below the optimum pH (i.e. carboxylic groups of histidine - $pK_a$ around 3). The side-chain of histidine ($pK_a$ around 6) offered a convenient buffering capacity to maintain the pH at the value required.

Example 3 - Papain

Papain consists of one unit ($M_r$ of each approx. 21,000). The isoelectric point of papain is approximately 8.7. Papain is a protease and if activated it is capable of self-digestion. To avoid self-digestion it has to be kept in inactivated (oxidised) form. All experiments involving papain presented here were carried out using the inactive (oxidised) form of papain.

The abundance of the acid-base amino acids in each subunit is shown in Table 6.

**Table 6**

| Amino acid | Total number of side-chains in the subunit | Number of side-chains exposed at the subunit surface |
|---|---|---|
| Aspartic acid (D) | 6 | 3 |
| Glutamic acid (E) | 10 | 7 |
| Histidine (H) | 2 | 0 |
| Cysteine (C) | 7 | 4 |
| Tyrosine (Y) | 19 | 10 |
| Lysine (K) | 10 | 8 |
| Arginine (R) | 12 | 11 |

The proton exchange frequency profile for papain is shown in Fig. 10. The pH optimisation algorithm gives the following results for papain:

$$P_{minimum} = 0.54 \text{ (at pH 6.3)}$$

$$X = 0.33$$

$$Y = 0.87$$

Estimated pH optimum = 5.8

Papain solutions, both fresh and after incubation at increased temperature, were assayed for papain activity. This was performed according to the following procedure:

100 $\mu$l of the papain sample was mixed with 100 $\mu$L of cysteine (24 mg/mL prepared in 25 mM phosphate buffer, pH 6.9). 160 $\mu$L of EDTA (2.5 mM prepared in 250 mM phosphate buffer, pH 6.0) was added and the resulting mixture was incubated at 60°C for 10 min. 160 $\mu$L of BANA (5 mg/mL prepared in 20% DMSO/80% water) was added and incubated at 60°C for another 10 min. The reaction was stopped by addition of 280 $\mu$l of HCl/methanol

mixture (prepared by mixing 1 mL of 5 M HCl and 9 mL of methanol). 400 $\mu$L of DMAC was added and the final mixture was allowed to stand at room temperature for 25 min. Absorbance of the mixture was then measured at 540 nm. If the colour intensity was too great to allow an accurate reading, the sample was diluted with a defined volume of 80% (v/v) methanol (prepared by mixing 4 volume parts of methanol and 1 volume part of deionised water) to bring the colour back on scale. The results were expressed as percentage recovery, by reference to the absorbance measured in the fresh samples (i.e. prior to incubation at increased temperature).

Example 3.1: Poor activity recovery on incubation of papain outside of the calculated pH optimum in the absence of beneficial additives

The effect of 50 mM citrate buffer ($pK_{a1}$ = 4.8, $pK_{a2}$ = 6.4) and of TRIS buffer on the stability of papain at 65°C was investigated at pH outside of the estimated pH optimum for papain. Citrate buffer was prepared by mixing citric acid (50 mM) with sodium hydroxide (5 M) to achieve the required pH. TRIS buffer was prepared by mixing Tris base (50 mM) with hydrochloric acid (5 M) to achieve required pH. The papain activity dropped to about 20% in 4 hours and almost to zero in 22 hours of incubation at 65°C. The recovery of the activity was poor because the pH was outside of the calculated pH optimum (in spite of the presence of the "beneficial additive" in the case of TRIS).

Example 3.2: Satisfactory activity recovery on incubation of papain near the calculated pH optimum in the presence of beneficial additives

Incorporation of glutamate and histidine into the papain formulation adjusted to the near-optimum pH resulted in satisfactory preservation of papain activity on incubation at 65°C. The background solution was prepared by mixing glutamic acid (50 mM) and histidine (50 mM) to achieve the required pH. Over 65% of the original activity was still measurable after 22 h incubation at 65°C if the pH was adjusted to 5.7. The decline of the enzyme activity was only slightly more rapid using the same formulations at pH 5.4 and 6.1. Nevertheless, using the same formulation with pH adjusted to >0.3 unit away from the optimum resulted in considerably faster decline of the enzyme activity. The formulation contained a positively charged protonatable group with $pK_a$ above the optimum pH (i.e. amino group of the two amino acids - $pK_a$ around 9) and a negatively charged protonatable group with $pK_a$ below the optimum pH (i.e. carboxylic groups of the tw amino acids - $pK_a$ around 3). The side-chain of histidine ($pK_a$ around 6) offered a convenient buffering capacity to maintain the pH at the value required.

Example 4 - Glutamate dehydrogenase (from bovine liver)

Glutamate dehydrogenase (from bovine liver) consists of six subunits ($M_r$ of each approx. 56,000). The isoelectric point of glutamate dehydrogenase is approximately 5.5. The abundance of the acid-base amino acids in each subunit is shown in Table 7.

**Table 7**

| Amino acid | Total number of side-chains in the subunit | Number of side-chains exposed at the subunit surface |
|---|---|---|
| Aspartic acid (D) | 29 | 22 |
| Glutamic acid (E) | 31 | 27 |
| Histidine (H) | 13 | 7 |
| Cysteine (C) | 6 | 0 |
| Tyrosine (Y) | 18 | 6 |
| Lysine (K) | 30 | 25 |
| Arginine (R) | 30 | 20 |

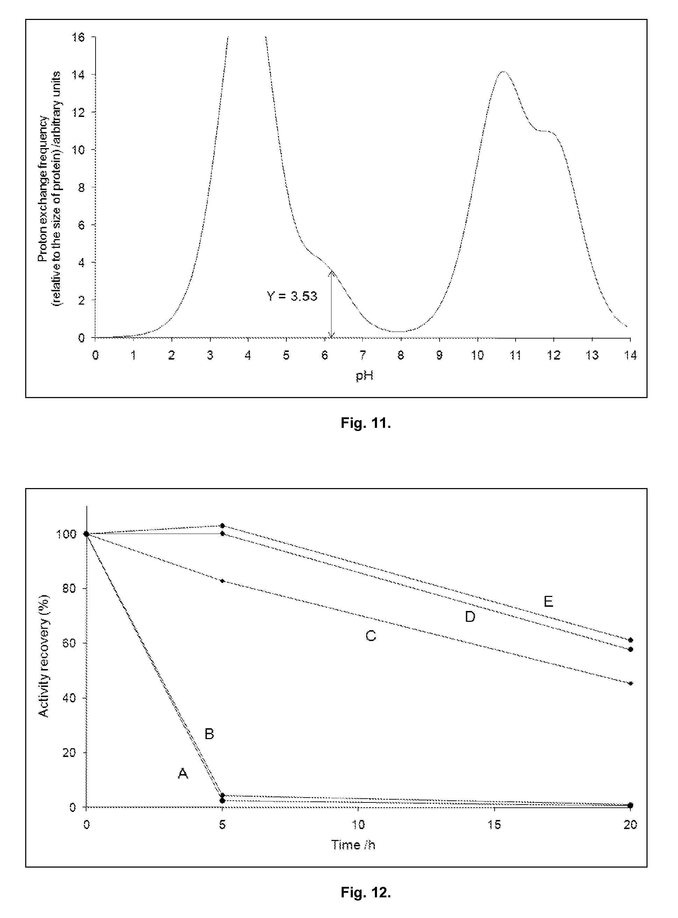

The proton exchange frequency profile for glutamate dehydrogenase is shown in Fig. 11. The pH optimisation algorithm gives the following results for glutamate dehydrogenase:

$$P_{minimum} = 0.32 \text{ (at pH 7.9)}$$

$$X = 3.21$$

$$Y = 3.53$$

Estimated pH optimum = 6.2

Glutamate dehydrogenase solutions, both fresh and after incubation at increased temperature, were assayed for glutamate dehydrogense activity. This was performed according to the following procedure:

100 $\mu$L of the glutamate dehydrogenase sample was added to a cuvette containing the mixture of the following reagents:

- 2.5 mL of phosphate buffer (0.2 M, pH 7.4) containing EDTA (350 $\mu$M)
- 200 $\mu$L of 2-oxyglutaric acid (200 mM)
- 100 $\mu$L of ammonium sulphate (1 M)
- 100 $\mu$L of NADH (7 mM)

These were mixed together quickly. Time = 0 was counted from the addition of the glutamate dehydrogenase sample. Precisely after 5 min, the absorbance was then read at 340 nm. The results were expressed as percentage recovery, by reference to the absorbance measured in the samples prior to their incubation at increased temperature.

Example 4.1: Poor activity recovery on incubation of glutamate dehydrogenase outside of the calculated pH optimum in the absence of beneficial additives

The effect of 50 mM citrate buffer ($pK_{a1}$ = 4.8, $pK_{a2}$ = 6.4) and of TRIS buffer on the stability of glutamate dehydrogenase at 55°C was investigated at pH outside of the estimated pH optimum for glutamate dehydrogenase. Citrate buffer was prepared by mixing citric acid (50 mM) with sodium hydroxide (5 M) to achieve the required pH. TRIS buffer was prepared by mixing Tris base (50 mM) with hydrochloric acid (5 M) to achieve required pH. The enzyme activity dropped to about 15% (in the case of TRIS buffer, pH 8.0) and to almost zero (in the case of citrate buffer (pH 4.0) after 4 hours of incubation at 55°C. The activity fell to zero in TRIS buffer after 22 hours of incubation at the increased temperature. The recovery of the activity was poor because the pH was outside of the calculated pH optimum (in spite of the presence of the "beneficial additive" in the case of TRIS).

Example 4.2: Satisfactory activity recovery on incubation of glutamate dehydrogenase near the calculated pH optimum in the presence of beneficial additives

Incorporation of lysine (50 mM) into the glutamate dehydrogenase formulation adjusted to the near-optimum pH resulted in satisfactory preservation of the enzyme activity on incubation at 55°C. The background solution was prepared by mixing 9 parts of lysine (55 mM) with 1 part of disodium hydrogen orthophosphate (50 mM). The pH was adjusted to the required value with citric acid (1 M). Over 75% of the original activity was still measurable after 22 h incubation at 55°C if the pH was adjusted to 6.1. The decline of the enzyme activity was only slightly more rapid using the same formulations at pH 5.8 and 6.4. Nevertheless, using the same formulation with pH adjusted to >0.3 unit away from the optimum resulted in considerably faster decline of the enzyme activity. The formulation contained a positively charged protonatable group with $pK_a$ above the optimum pH (i.e. amino group of lysine - $pK_a$ around 9) and a negatively charged protonatable group with $pK_a$ below the optimum pH (i.e. carboxylic groups of lysine - $pK_a$ around 3). 10 mM citrate phosphate buffer was used to maintain the pH.

Example 5 - Horseradish peroxidase

Horseradish peroxidase consists of one unit ($M_r$ approx. 42,000). The commonly stated value for the most abundant isoform of peroxidase in the horseradish root is approximately 8.6. The abundance of the acid-base amino acids in the horseradish peroxidase unit is shown in Table 8.

**Table 8**

| Amino acid | Total number of side-chains in the subunit | Number of side-chains exposed at the subunit surface |
|---|---|---|
| Aspartic acid (D) | 21 | 9 |
| Glutamic acid (E) | 7 | 5 |
| Histidine (H) | 3 | 1 |
| Cysteine (C) | 0(8)* | 0 |
| Tyrosine (Y) | 5 | 2 |
| Lysine (K) | 6 | 4 |
| Arginine (R) | 20 | 16 |
| * The first number indicates the number of cysteines that are not engaged in disulphide bond. The number in brackets indicates the total number of cysteines in the subunit. | | |

The proton exchange frequency profile for horseradish peroxidase is shown in Fig. 12. The pH optimisation algorithm gives the following results for horseradish peroxidase:

$$P_{minimum} = 0.08 \text{ (at pH 8.1)}$$

$$X = 0.42$$

$$Y = 0.49$$

Estimated pH optimum = 7.1

Horseradish peroxidase solutions, both fresh and after incubation at increased temperature, were assayed for horseradish peroxidase activity. This was performed according to the following procedure:

10 $\mu$L of the horseradish peroxidase sample was added to a cuvette containing the mixture of the following reagents:

- 2.5 mL of citrate/phosphate buffer (0.05 M, pH 5.0)
- 100 $\mu$L of hydrogen peroxide (2 mM)
- 100 $\mu$L of TMB (3 mg /mL, dissolved in DMSO)

These were mixed together quickly. Time = 0 was counted from the addition of the horseradish peroxidase sample. Precisely after 3 min, the absorbance was then read at 630 nm. The results were expressed as percentage recovery, by reference to the absorbance measured in the samples prior to their incubation at increased temperature.

Example 5.1: Poor activity recovery on incubation of horseradish peroxidase outside of the calculated pH optimum in the absence of beneficial additives

The effect of 50 mM citrate/phosphate buffer (pH 5.0) and of phosphate buffer (pH 6.0) on the stability of horseradish peroxidase at 65°C was investigated at pH outside of the estimated pH optimum for horseradish peroxidase. Citrate/Phosphate buffer was prepared by mixing citric acid (50 mM) with disodium hydrogen orthophosphate (50 mM) to achieve the required concentration. The phosphate buffer was prepared by mixing disodium hydrogen orthophosphate (50 mM) and sodium dihydrogen orthophosphate (50 mM) to achieve the required pH. The enzyme activity dropped to < 10% in the case of citrate/phosphate buffer (pH 5.0) and to about 30% in the case of phosphate buffer (pH 6.0) after 22 hours of incubation at 65°C. The recovery of the activity was poor because the pH was outside of the calculated pH optimum. There was also no positively charged protonatable functional group.

<u>Example 5.2: Satisfactory activity recovery on incubation of horseradish peroxidase near the calculated pH optimum in the presence of beneficial additives</u>

Incorporation of lysine and lactate into the horseradish peroxidase formulation adjusted to the near-optimum pH resulted in satisfactory preservation of the enzyme activity on incubation at 65°C. The background solution was prepared by mixing lysine (50 mM) with lactate (50 mM) to achieve the required pH. Almost 75% of the original activity was still measurable after 22 h incubation at 65°C if the pH was adjusted to 7.2 or 7.5. The decline of the enzyme activity was only slightly more rapid using the same formulations between the pH 6.9 and 8.1. This makes horseradish peroxidase (together with lactoperoxidase as shown in Example 6) an enzyme with the broadest pH optimum compared with other enzymes studied. This could be explained by the fact that the pH optimum for the enzyme is located in a relatively broad minimum in the proton exchange frequency graph (see Fig. 11).

<u>Example 6 - Lactoperoxidase</u>

The isoelectric point of lactoperoxidase quoted in literature is 8.0-9.2. If it can be assumed that there is a structural similarity between horseradish peroxidase and lactoperoxidase (as the similar value of the isoelectric points might indicate), then the estimated pH optimum for lactoperoxidase should be similar to that for horseradish peroxidase.
Lactoperoxidase solutions, both fresh and after incubation at increased temperature, were assayed for lactoperoxidase activity. This was performed according to the following procedure:

100 $\mu$L of the lactoperoxidase sample was mixed with 10 mL of deionised water. 1.25 mL of the resulting mixture was added to a cuvette containing the mixture of the following reagents:

- 1.25 mL of citrate/phosphate buffer (0.1 M, pH 5.0)
- 100 $\mu$L of hydrogen peroxide (2 mM)
- 100 $\mu$L of TMB (3 mg /mL, dissolved in DMSO)

These were mixed together quickly. Time = 0 was counted from the addition of the lactoperoxidase sample. Precisely after 5 min, the absorbance was then read at 630 nm. The results were expressed as percentage recovery, by reference to the absorbance measured in the samples prior to their incubation at increased temperature.

<u>Example 6.1: Poor activity recovery on incubation of lactoperoxidase outside of the calculated pH optimum in the absence of beneficial additives</u>

The effect of 50 mM citrate/phosphate buffer (pH 5.0) and of phosphate buffer (pH 6.0) on the stability of lactoperoxidase at 65°C was investigated at pH outside of the estimated pH optimum for lactoperoxidase (as calculated from horseradish peroxidase sequence). Citrate/Phosphate buffer was prepared by mixing citric acid (50 mM) with disodium hydrogen orthophosphate (50 mM) to achieve the required concentration. The phosphate buffer was prepared by mixing disodium hydrogen orthophosphate (50 mM) and sodium dihydrogen orthophosphate (50 mM) to achieve the required pH. The enzyme activity dropped to almost zero in the case of citrate/phosphate buffer (pH 5.0) and to about 10% in the case of phosphate buffer (pH 6.0) after 4 hours of incubation at 65°C. The recovery of the activity was poor because the pH was outside of the calculated pH optimum. There was also no positively charged protonatable functional group.

<u>Example 6.2: Satisfactory activity recovery on incubation of lactoperoxidase near the calculated pH optimum in the presence of beneficial additives</u>

Incorporation of TRIS into the lactoperoxidase formulation adjusted to the near-optimum pH (as calculated from horseradish peroxidase sequence) resulted in satisfactory preservation of the enzyme activity on incubation at 65°C. The background solution was prepared by mixing TRIS base (50 mM) with hydrochloric acid (5 M) to achieve the required pH. 100 % of the original activity was still measurable after 4 h incubation at 65°C if the pH was adjusted to 7.8 or 8.2. There was only a small decline of the enzyme activity using the same formulations at pH 7.4 and 8.6.

<u>Example 7 - Uricase</u>

Uricase solutions (typically 250 $\mu$g mL$^{-1}$), both fresh and after incubation at increased temperature, were assayed for uricase activity. This was performed according to the following procedure:

The following solutions were mixed in a 1 cm cuvette:

- 1.5 mL of borate buffer (25 mM, pH 8.5); prepared by adjusting the pH of 25 mM boric acid using sodium hydroxide
- 0.8 mL of sodium urate (2 mM)

40 $\mu$L of uricase sample (max. 250 $\mu$g mL$^{-1}$ of 5.3 U mg$^{-1}$ preparation) was added and mixed quickly. Time = 0 was counted from the addition of the uricase. After 5 min, the following reagents were added in this particular order (the first reagent should be added at exactly 5 min, the timing of the other reagents addition is less crucial):

- 0.8 mL of citrate/phosphate buffer (0.5 M, pH 4.0); prepared by mixing 0.5 M citric acid with 0.5 M disodium hydrogen phosphate to achieve the pH required
- 100 $\mu$L of TMB (3 mg mL$^{-1}$, dissolved in DMSO)
- 100 •L of lactoperoxidase (1 mg mL$^{-1}$, dissolved in water)

The resulting solution was mixed thoroughly and absorbance was read at 630 nm using a Unicam UV-visible spectrophotometer (type: Helios gamma). The results were expressed as percentage recovery, by reference to the absorbance measured in the fresh samples (i.e. prior to incubation at increased temperature).

The activity of uricase decreased very rapidly on incubation at 60°C in control solutions (phosphate buffer, pH 7.0; TRIS/HCl buffer, pH 7.2). Considerably better recovery was achieved in borate buffer (pH 9.0). However, the best stability was observed if all conditions of the protein stabilising theory were met (i.e. if both the positively and the negatively charged ionisable groups were present at the optimum pH). This is demonstrated in Fig. 12 for the mixture of purine and succinate and TRIS and serine (see Fig. 12.

Example 8 - Hepatitis B recombinant vaccine

The *in vitro* antigenic activity of the Hepatitis B vaccine was measured using the AUSZYME monoclonal diagnostic kit (Abbott Laboratories; cat no. 1980-64). The antigenic activity was determined both in the whole vaccine and in the supernatant following centrifugation (13,000 RPM, 5 min). Each sample was measured in triplicate. The antigenic activity was expressed as a percentage with respect to the value measured of the untreated refrigerated vaccine as follows:

$$ R = \frac{(S - N)}{(C - N)} \times 100 $$

$$ S = \frac{S1 + S2 + S3}{3} $$

$$ C = \frac{C1 + C2 + C3}{3} $$

where:

R is the recovery of antigenic activity (%)
N is the value of the negative control AUSZYME measurement
C1, C2 and C3 are the values of three repeated AUSZYME measurements of the Control sample (i.e. untreated refrigerated vaccine)
S1, S2 and S3 are the values of three repeated AUSZYME measurements of the tested sample.

The remaining antigenic activity of Hepatitis B vaccine was tested after incubation at 55°C for 2, 4 and 7 weeks. The stabilising formulations consisting of histidine and phosphate anion and adjusted to the optimum pH retained >95% of the original antigenic activity after 7 weeks. In contrast, the remaining antigenic activity of the original vaccine formulation (18 mM phosphate buffer, pH 6.9-7.1, containing 132 mM sodium chloride) was < 10% at this point. The stabilising formulations did not appear to affect the alumina-antigen association as the antigenic activity measured in the supernatant of the stabilising formulation following centrifugation was comparable with that measured in the supernatant of the original sample. It is believed that the presence of phosphate anion ensures optimal binding of the vaccine onto the alumina.

Example 9 - Effect of polyalcohols

A beneficial effect of a selection of polyalcohols was demonstrated on the stability of glucose oxidase at 60°C in the presence of the optimised proton-exchange excipients at optimised pH. Using the optimised mixture of histidine (25 mM) and lactate (25 mM) at pH 5.3 as the background, the recovery of glucose oxidase activity after 20 hours of incubation at 60°C was better in the presence of inositol, lactitol, mannitol, xylitol and trehalose (all at 10 %) than in the absence of these polyalcohols.

A similar effect on glucose oxidase stability was observed of another polyalcohol, glycerol. The effect of glycerol was studied at various concentrations and it was found that the effect was most pronounced at concentrations between 10% and 20% (w/w). Nevertheless, even the presence of 1.33% concentration glycerol resulted in measurable improvement of glucose oxidase stability over that achieved in the optimised histidine/lactate mixture (pH 5.3).

Importantly, the presence of polyalcohols was found not to have a significant beneficial effect if the background solution was away from the optimum conditions in terms of the presence of the proton-exchange excipients and the pH. Thus, for example, if phosphate buffer (50 mM, pH 7.0) was used as the background solution then incubation of glucose oxidase at 60°C for 20 hours resulted in a complete loss of glucose oxidase activity both in the presence and in the absence of the selected sugar alcohols.

Example 10 - Glucose oxidase adsorbed on alumina particles

Glucose oxidase was adsorbed onto alumina particles by incubation of alumina in a solution of glucose oxidase (25 $\mu$g/mL) at 4°C overnight. The activity of the adsorbed glucose oxidase, both fresh and after incubation at 40°C, was assayed according to the following procedure:

The following solutions were mixed in a 1 cm cuvette:

- 2.0 mL of Citrate/Phosphate buffer (50 mM, pH 5.0); prepared by mixing citric acid (50 mM) with disodium hydrogen orthophosphate (50 mM) to achieve the required pH.
- 40% (w/w) glucose in DI water
- 100 $\mu$L Lactoperoxidase (1 mg/mL) in DI water
- 100 $\mu$L of TMB (3 mg /mL, dissolved in DMSO)

10 $\mu$L of glucose oxidase sample was added and mixed quickly. Time = 0 was counted from the addition of the the sample. Absorbance was read at 630 nm exactly 5 min after addition of sample using a Unicam UV-visible spectrophotometer (Type: Helios gamma). The results were expressed as percentage recovery, by reference to the absorbance measured in the fresh samples (i.e. prior to incubation at increased temperature).

The optimised formulation that was found previously to stabilise glucose oxidase in solution (Histidine + Glutamate, total of 50 mM, pH 5.0) was also found to be effective in preserving the glucose oxidase activity when adsorbed on alumina particles. Unlike in the aqueous solutions, the efficiency of the formulation was dependent on the concentration of the excipients. Thus, the formulation containing the total of 50 mM excipients resulted in better activity recovery than that containing the total of 10 mM excipients. The recovery was considerably better than that observed in DI water or phosphate buffer (50 mM, pH 7.0).

Example 11 - Effect of polyalcohols

A beneficial effect of a selection of polyalcohols was demonstrated on the stability of catalase at 55°C in the presence of the optimised proton-exchange excipients at optimised pH. Using the optimised mixture of histidine (50 mM) and lactate (50 mM) at pH 6.6 as the background, the recovery of glucose oxidase activity after 24 hours of incubation at 55°C was better in the presence of inositol, lactitol, mannitol, xylitol and trehalose (all at 10%) than in the absence of these polyalcohols.

Example 12

HPLC assay was conducted as follows: Mobile phase A consisted of 0.1% TFA in water, mobile phase B consisted of 0.1 % TFA in acetonitrile. The mobile phases were filtered prior to their use. Linear gradient was employed: A/B (70:30) to A/B (68:32) in 20 min. The liquid chromatograph (Agilent 1100 series) was equipped with a 214 nm detector and a $4.6 \times 250$ mm column (Zorbax Eclipse XDB-C18) 00G-4167-E0) packed with octadecylsilyl silica gel with a granulometry of 5 $\mu$m, maintained at 30°C. The flow rate was maintained at 1 mL min$^{-1}$. 15 $\mu$L of aqueous samples of human insulin (typically 1-2.5 mg mL$^{-1}$) were injected.

The recovery of structural integrity of human insulin was studied in an accelerated storage trial on incubation at 65°C for 5 hours. Whilst the area of the main chromatographic peak dropped to about 50% in the control solution (phosphate buffer, 50mM, pH 7.0), more than 90% of insulin was recovered in the solution in the stabilised formulation comprising glutamate (25 mM) and lysine (25 mM) at pH 5.3.

Example 13-Human growth hormone

A HPLC assay was conducted as follows: Mobile phase was prepared by mixing 71 parts (by volume) of a solution of TRIS (0.05 M, in water adjusted with hydrochloric acid to a pH of 7.5) and 29 parts (by volume) of n-propylalcohol. The mobile phase was filtered prior to its use. The liquid chromatograph (Agilent 1100 series) was equipped with a 214 nm detector and a $4.6 \times 250$ mm column (Phenomenex 00G-4167-E0) packed with butylsilyl silica gel with a granulometry of 5 $\mu$m and a porosity of 30 nm, maintained at 45°C. The flow rate was maintained at 0.5 mL min$^{-1}$. 15 $\mu$L of aqueous samples of human growth hormone (typically 1-2.5 mg mL$^{-1}$) were injected.

The recovery of structural integrity of human growth hormone was studied in an accelerated storage trial on incubation at 40°C for 4 weeks. Whilst the area of the main chromatographic peak dropped to <35% in the control solutions (i.e. in phosphate buffer, 50mM, pH 7.0 or in water) more than 70% of human growth hormone was recovered in the solution in the stabilised formulations comprising glutamate (10 mM, ph 6.0) or lysine (10 mM, pH 6.0).

Aspects of the invention are defined by the following numbered paragraphs:

1. An aqueous system comprising a protein and one or more stabilising agents, **characterised in that**

(i) the one or more stabilising agents have ionisable groups capable of exchanging protons with the protein and with the ionised products of water dissociation;
(ii) the ionisable groups include first groups that are positively charged when protonated and uncharged when deprotonated, and second groups that are uncharged when protonated and negatively charged when deprotonated; and
(iv) the pH of the composition is within a range of protein stability that is at least 50% of the maximum stability of the protein with respect to pH.

2. An aqueous system comprising a protein and one or more stabilising agents, **characterised in that**

(i) the one or more stabilising agents have ionisable groups capable of exchanging protons with the protein and with the ionised products of water dissociation;
(ii) the ionisable groups include first groups that are positively charged when protonated and uncharged when deprotonated, and second groups that are uncharged when protonated and negatively charged when deprotonated; and
(iv) the pH of the composition is within a range of $\pm 0.5$ pH units of the pH at which the composition has maximum stability with respect to pH.

3. A system according to paragraph 1, wherein said range is at least 60% of the maximum stability.
4. A system according to paragraph 1, wherein said range is at least 70% of the maximum stability.
5. A system according to paragraph 1, wherein said range is at least 80% of the maximum stability.
6. A system according to any of paragraphs 1 to 5, which comprises one stabilising agent having said ionisable groups.
7. A system according to any of paragraphs 1 to 5, which comprises two stabilising agents respectively having said first and second ionisable groups.
8. A system according to any preceding paragraph, wherein the first and second groups have pKa values respectively higher and lower than the pH of the composition and are at least 50% of these groups are ionised.
9. A system according to paragraph 8, wherein at least 80% of the groups are ionised.
10. A system according to paragraph 8, wherein the groups are substantially completely ionised.
11. A system according to any of paragraphs 8 to 10, wherein the respective pKa values are each within 0.5 to 4 pH units of the pH of the composition.

12. A system according to paragraph 11, wherein the respective pKa values are each within 1 to 3 pH units of the pH of the composition.

13. A system according to any preceding paragraph, whose pH is 4 to 9.

14. A system according to any preceding paragraph, which additionally comprises a polyalcohol.

15. A system according to paragraph 13, which comprises at least 0.5% (w/w) of the polyalcohol.

16. A system according to any preceding paragraph, which comprises at least 0.1% (w/w) of the one or more stabilising agents.

17. A system according to paragraph 16, which comprises at least 0.5% (w/w) of the one or more stabilising agents.

18. A system according to any preceding paragraph, which comprises up to 200 mM of each stabilising agent.

19. A system according to any preceding paragraph, which comprises up to 100 mM of each stabilising agent.

20. A system according to any preceding paragraph, wherein the protein is in the native state.

21. A system according to any preceding paragraph, wherein the protein stability is measured in terms of retention of its functional and/or structural characteristics.

22. A system according to any preceding paragraph, wherein the protein is a hormone or growth factor.

23. A system according to any preceding paragraph, wherein the protein is a therapeutic enzyme.

24. A system according to any preceding paragraph, wherein the protein is a therapeutic antibody.

25. A system according to any preceding paragraph, wherein the protein is an interferon.

26. A system according to any preceding paragraph wherein the protein is immunogenic.

27. A system according to any preceding paragraph, which is an aqueous solution, suspension or dispersion.

28. A composition which comprises a protein and one or more stabilising agents as defined in any of paragraphs 1 to 26, adsorbed on a solid.

29. A composition according to paragraph 28, wherein the solid is a vaccine adjuvant such as alumina.

30. A composition according to paragraph 28 or paragraph 29, wherein the protein is immunogenic, for use as a vaccine.

31. A composition according to any of paragraphs 28 to 31, which additionally comprises phosphate.

32. A system or composition according to any preceding paragraph, for use in therapy or diagnosis practised on the human or animal body.

33. A sealed container containing a system or composition according to any preceding paragraph.

**Claims**

1. An aqueous system comprising a protein and two or more stabilising agents, **characterised in that**

   (i) the two or more stabilising agents have ionisable groups capable of exchanging protons with the protein and with the ionised products of water dissociation;
   (ii) wherein a first stabilising agent has first ionisable groups that are positively charged when protonated and uncharged when deprotonated, and a second stabilising agent has second ionisable groups that are uncharged when protonated and negatively charged when deprotonated, wherein the first and second ionisable groups have pKa values respectively 1 to 5 units higher and 1 to 5 units lower than the pH of the composition, and wherein the second stabilising agent is a carboxylic acid other than an amino acid; and
   (iii) the pH of the composition is within a range of protein stability that is at least 50% of the maximum stability of the protein with respect to pH.

2. An aqueous system comprising a protein and two or more stabilising agents, **characterised in that**

   (i) the two or more stabilising agents have ionisable groups capable of exchanging protons with the protein and with the ionised products of water dissociation;
   (ii) wherein a first stabilising agent has first ionisable groups that are positively charged when protonated and uncharged when deprotonated, and a second stabilising agent has second ionisable groups that are uncharged when protonated and negatively charged when deprotonated, wherein the first and second ionisable groups have pKa values respectively 1 to 5 units higher and 1 to 5 units lower than the pH of the composition, and wherein the second stabilising agent is a carboxylic acid other than an amino acid; and
   (iii) the pH of the composition is within a range of $\pm 0.5$ pH units of the pH at which the composition has maximum stability with respect to pH.

3. A system according to claim 1, wherein said range is at least 70% of the maximum stability.

4. A system according to any preceding claim, wherein at least 80% of the first and second ionisable groups are ionised.

5. A system according to claim 4, wherein the groups are substantially completely ionised.

6. A system according to any preceding claim, wherein the respective pKa values are each within

> (i) 1 to 4 pH units; or
> (ii) 1 to 3 pH units

of the pH of the composition.

7. A system according to any preceding claim, wherein the second stabilising agent is selected from formic acid, glyoxylic acid, oxalic acid, acetic acid, glycolic acid, pyruvic acid, malonic acid, lactic acid, glyceric acid, fumaric acid, succinic acid, malic acid, $\alpha$-tartaric acid, glutaric acid, ascorbic acid, adipic acid and gallic acid.

8. A system according to claim 7, wherein the second stabilising agent is lactic acid.

9. A system according to any preceding claim, wherein the first stabilising agent is an amine.

10. A system according to any preceding claim, whose pH is 4 to 9.

11. A system according to any preceding claim, which comprises at least 0.1 % (w/w) of the one or more stabilising agents.

12. A system according to any preceding claim, which comprises up to 200 mM of each stabilising agent.

13. A system according to any preceding claim, wherein the protein is a hormone, a growth factor, a therapeutic enzyme a therapeutic antibody or an interferon, or. wherein the protein is immunogenic.

14. A system according to any preceding claim, which is an aqueous solution.

15. A composition comprising protein in solid state obtainable by partially or fully removing water from an aqueous system according to any one of claims 1 to 14.

Fig. 1.

Fig. 2.

Fig. 3.

Fig. 4.

**Fig. 5.**

**Fig. 6**

**Fig. 7.**

**Fig. 8.**

**Fig. 9**

**Fig. 10.**

**Fig. 11.**

**Fig. 12.**

| | Europäisches Patentamt European Patent Office Office européen des brevets | **EUROPEAN SEARCH REPORT** | Application Number EP 10 17 5575 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 03/009869 A (CHIRON SPA; CONTORNI, MARIO; MAFFEI, MASSIMO) 6 February 2003 (2003-02-06) * claims 1-13,19 * * examples 1,2,8 * * page 7 * ----- | 1-15 | INV. C12N9/96 A61K39/39 |
| X | EP 1 314 437 A (CHUGAI SEIYAKU KABUSHIKI KAISHA) 28 May 2003 (2003-05-28) * claims 1-35 * * page 5 - page 6 * * examples 1-6 * ----- | 1-15 | |
| A | ELCOCK A H: "Realistic modeling of the denatured states of proteins allows accurate calculations of the ph dependence of protein stability" JOURNAL OF MOLECULAR BIOLOGY, LONDON, GB, vol. 294, no. 4, 10 December 1999 (1999-12-10), pages 1051-1062, XP004461830 ISSN: 0022-2836 * the whole document * ----- | 1-15 | |
| A | ALEXOV EMIL: "Numerical calculations of the pH of maximal protein stability. The effect of the sequence composition and three-dimensional structure." EUROPEAN JOURNAL OF BIOCHEMISTRY, vol. 271, no. 1, January 2004 (2004-01), pages 173-185, XP002398496 ISSN: 0014-2956 * the whole document * ----- -/-- | 1-15 | TECHNICAL FIELDS SEARCHED (IPC) C12N A61K G06F |

The present search report has been drawn up for all claims

| Place of search | Date of completion of the search | Examiner |
|---|---|---|
| Munich | 29 September 2010 | Bayer, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...............................................................................

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 10 17 5575

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ANTOSIEWICZ JAN ET AL: "Prediction of pH-dependent properties of proteins" JOURNAL OF MOLECULAR BIOLOGY, vol. 238, no. 3, 1994, pages 415-436, XP002398497 ISSN: 0022-2836 * the whole document * | 1-15 | |
| A | WO 03/021258 A (BOARD OF REGENTS, THE UNIVERSITY OF TEXAS SYSTEM; HILSER, VINCE; WHITT) 13 March 2003 (2003-03-13) * page 13 - page 14 * * examples 2-4,6 * * figure 4 * | 1-15 | |

TECHNICAL FIELDS
SEARCHED (IPC)

The present search report has been drawn up for all claims

| Place of search | Date of completion of the search | Examiner |
|---|---|---|
| Munich | 29 September 2010 | Bayer, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 10 17 5575

This annex lists the patent family members relating to the patent documents cited in the above-mentioned European search report.
The members are as contained in the European Patent Office EDP file on
The European Patent Office is in no way liable for these particulars which are merely given for the purpose of information.

29-09-2010

| Patent document cited in search report | | Publication date | Patent family member(s) | | Publication date |
|---|---|---|---|---|---|
| WO 03009869 | A | 06-02-2003 | AU | 2002330681 B2 | 22-05-2008 |
| | | | CA | 2452720 A1 | 06-02-2003 |
| | | | CN | 1535158 A | 06-10-2004 |
| | | | EP | 1409013 A1 | 21-04-2004 |
| | | | RU | 2323002 C2 | 27-04-2008 |
| EP 1314437 | A | 28-05-2003 | AU | 7778101 A | 25-02-2002 |
| | | | WO | 0213860 A1 | 21-02-2002 |
| | | | US | 2003190316 A1 | 09-10-2003 |
| WO 03021258 | A | 13-03-2003 | CA | 2459095 A1 | 13-03-2003 |
| | | | EP | 1438581 A1 | 21-07-2004 |
| | | | JP | 2005502945 T | 27-01-2005 |

EPO FORM P0459

For more details about this annex : see Official Journal of the European Patent Office, No. 12/82

**EP 2 264 161 A1**

REFERENCES CITED IN THE DESCRIPTION

**Patent documents cited in the description**

- EP 0513914 A

- US 3051627 A

**Non-patent literature cited in the description**

- **Iyer S. et al.** *Vaccine,* 2004, vol. 22, 1475-1479